(19)

**Europäisches Patentamt**
**European Patent Office**
**Office européen des brevets**

(11)  **EP 4 681 722 A1**

(12)  **EUROPEAN PATENT APPLICATION**

(43) Date of publication:
    **21.01.2026   Bulletin 2026/04**

(21) Application number: **24306213.0**

(22) Date of filing: **18.07.2024**

(51) International Patent Classification (IPC):
    *A61K 38/05* (2006.01)    *A61K 31/198* (2006.01)
    *A61K 31/7105* (2006.01)    *A61N 5/00* (2006.01)
    *A61P 17/00* (2006.01)

(52) Cooperative Patent Classification (CPC):
    (C-Sets available)
    **A61K 38/05; A61K 31/198; A61K 31/7105;**
    **A61N 5/0616; A61P 17/00;** A61N 2005/0661

(Cont.)

(84) Designated Contracting States:
    **AL AT BE BG CH CY CZ DE DK EE ES FI FR GB**
    **GR HR HU IE IS IT LI LT LU LV MC ME MK MT NL**
    **NO PL PT RO RS SE SI SK SM TR**
    Designated Extension States:
    **BA**
    Designated Validation States:
    **GE KH MA MD TN**

(71) Applicants:
    • **Institut National de la Santé et de la Recherche Médicale**
      **75013 Paris (FR)**
    • **Centre National de la Recherche Scientifique**
      **75016 Paris (FR)**

    • **Ecole Normale Supérieure de Lyon**
      **69007 Lyon (FR)**
    • **Université Claude Bernard Lyon 1**
      **69100 Villeurbanne (FR)**

(72) Inventor: **The designation of the inventor has not yet been filed**

(74) Representative: **Inserm Transfert**
    **PariSanté Campus**
    **10 rue d'Oradour-sur-Glane**
    **75015 Paris (FR)**

Remarks:
    The complete document including Reference Table(s) and the Sequence Listing(s) can be downloaded from the EPO website

(54)  **METHODS FOR THE TREATMENT OF INFLAMMATORY SKIN DISEASES WITH UROCANASE INHIBITOR**

(57)    In the present invention, inventors decipher the relationship between the skin microbiome and its ability to inhibit an important function of sunlight and UVB radiation, namely their ability to suppress local and systemic adaptive immune responses and induce tolerance to environmental antigens on the skin. The landscape of microbial communities inhabiting the skin, their ability to metabolize cis-urocanic acid (cis-UCA) a major UV photoproduct and reduce its immunomodulative properties in the standard model of induction of contact hypersensitivity (CHS) were examined. UVB and topically applied cis-UCA induced transient and significant perturbations in skin bacterial communities with increased abundance of several bacterial phyla such as Firmicutes and Bacteroidetes. Bioinformatic analysis showed that several of the increased species, such as Staphylococcus epidermidis, expressed the HutU gene, which encodes urocanase, the enzyme degrading UCA. Comparison of the contact hypersensitivity (CHS) response provided functional evidence that the presence of HutU+ bacteria limit the immunomodulation effect of cis-UCA. Moreover, topical application of the urocanase inhibitor, glycylglycine, restricted the metabolism of cis-UCA by HutU+ bacteria and restored immunomodulation. Accordingly a first object of the present invention relates to a method of treating an inflammatory skin disease in a subject in need thereof comprising administering to the subject a therapeutically effective amount of an urocanase inhibitor associated or not with UV phototherapy.

EP 4 681 722 A1

(52) Cooperative Patent Classification (CPC): (Cont.)

C-Sets
**A61K 38/05, A61K 2300/00**

**Description**

**FIELD OF THE INVENTION:**

The present invention relates to methods for the treatment of inflammatory skin diseases with an urocanase inhibitor associated or not with phototherapy.

**BACKGROUND OF THE INVENTION:**

The epidermis constitutes a physical and functional barrier against environmental agents, essential in maintaining skin homeostasis. The combination of inflammation and barrier dysfunction is evident in the pathogenesis of severe dermatitis such as atopic dermatitis (Palmer CNA, et al.. Nat Genet 2006;38:441-6. ; Cork MJ, et al. J Invest Dermatol 2009;129:1892-908). However little is known about how immunological dysregulation interact and contribute to the initiation and maintenance of such inflammatory diseases.

The skin hosts a vast ecosystem composed of millions of microorganisms, including bacteria, fungi, and viruses. The composition of the cutaneous microbiome is highly unique, complex and varies greatly depending on the anatomical location.

Commensal microbes adapt their metabolism to the resources available in their microenvironment. They feed on nutrients produced in the skin (dead corneocytes, extracellular matrix lipids, secretions from skin appendages) or that result from lifestyle habits (xenobiotics from skin care products, drugs). In turn, they produce a range of bioactive molecules, including short-chain fatty acids, lactic acid, tryptophan metabolites and antimicrobial compounds, that modify their microhabitat and host cell functions through microbe-microbe or microbe-host interactions (1). For example, well-known commensal species such as Cutibacterium acnes and Staphylococcus epidermis (S. epidermis) play a critical role in maintaining host skin physiology or promoting skin immunity (2, 3).

To date, many internal and external factors have been identified that influence the composition of the skin microbiome. These include various individual parameters such as race, gender, age, hormone levels, diet and hygiene, but environmental factors and the effects of occupation, pollution and climate also have a major influence (4).

The interactions between solar UV radiation and the skin microbiome are beginning to be studied. It is currently unclear whether UV exposure disrupts the healthy microbiome due to its ability to cause DNA damage or due to its widespread impact on skin immunity by stimulating the release of antimicrobial peptides and proinflammatory mediators from keratinocytes. In previous work, we showed that the skin microbiome inhibits an important effect of sunlight and UVB radiation, namely their ability to suppress local and systemic adaptive immune responses and to induce tolerance to environmental antigens on the skin (5). However, the mechanisms underlying this effect have yet to be elucidated (4).

In this study, the effects of UVB and one of its major photoproducts, cis-urocanic acid (cis-UCA), on the skin microbiome of mice were investigated. UCA occurs mainly as a trans-isomer in the stratum corneum and accounts for approximately 0.7% of the dry weight of the epidermis.(6) Upon exposure to UVB radiation, trans-UCA is isomerized to cis-UCA in a dose-dependent manner (7, 8), and the cis-isomer can be detected in serum and urine for several days thereafter (9, 10), Landmark studies have shown that cis-UCA is an important mediator of UV-induced immunomodulation (11, 12) and that treatment of irradiated mice with a monoclonal antibody specific for cis-UCA blocks the immunomodulatory effects of UV exposure (13, 14). Moreover, the topical application of cis-UCA in unexposed animals restores the immunomodulatory effects of UVB (15, 16).

Overall, inventors results show that UVB and cis-UCA stimulate the growth of UCA-degrading bacteria, which then regulate the immunomodulatory functions of cis-UCA, thereby providing a striking case study that demonstrates the ability of the skin microbiome to reshape host immune cell functions.

**SUMMARY OF THE INVENTION:**

The present invention relates to methods for the treatment of inflammatory skin diseases with an urocanase inhibitor associated or not with phototherapy.

In particular, a first object of the present invention relates to a method of treating an inflammatory skin disease in a subject in need thereof comprising administering to the subject a therapeutically effective amount of an urocanase inhibitor

In a particular embodiment said method is associated with phototherapy.

A second object of the present invention relates to an urocanase inhibitor for use in a treatment of an inflammatory skin disease.

In a particular embodiment said use being associated with phototherapy.

**DETAILED DESCRIPTION OF THE INVENTION:**

Loss of epidermal integrity is known to play a role in the pathogenesis of skin inflammatory disorders, especially those associate with UV exposure. However the intertwined mechanisms of immunological dysregulation should be clarified. The inventors decipher the relationship between the skin microbiome and its ability to inhibit an important function of sunlight and UVB radiation, namely their ability to suppress local and systemic adaptive immune responses and induce tolerance to environmental antigens on the skin.

To gain further insight into the underlying mechanisms, C57BL/6 mice were exposed to UVB or topically applied cis-urocanic acid (cis-UCA), a major UV photoproduct. The landscape of microbial communities inhabiting the skin, their ability to metabolize cis-UCA and reduce its immunomodulative properties in the standard model of induction of contact hypersensitivity (CHS) to 2,4-dinitrofluorobenzene were then examined.

UVB and cis-UCA induced transient and significant perturbations in skin bacterial communities with increased abundance of several bacterial phyla such as Firmicutes and Bacteroidetes. Bioinformatic analysis showed that several of the increased species, such as *Staphylococcus epidermidis*, expressed the HutU gene, which encodes urocanase, the enzyme degrading UCA. In vitro culture experiments demonstrated that HutU+ but not HutU- strains used cis-UCA for growth, which explains why the amount of cis-UCA available in the skin after UVB exposure was dramatically increased in mice with reduced bacterial load after disinfection with chlorhexidine.

Comparison of the contact hypersensitivity (CHS) response in mice with normal and disinfected skin or in gnotobiotic-like models colonized with HutU+/- strains provided functional evidence that the presence of HutU+ bacteria limit the immunomodulatory effect of cis-UCA. Moreover, topical application of the urocanase inhibitor, glycylglycine, restricted the metabolism of cis-UCA by HutU+ bacteria and restored immunomodulation.

Overall, these results provide mechanistic evidence that certain cutaneous bacterial species can metabolize the UVB mediator, cis-UCA, thus reducing its concentration, and thereby diminishing UV-induced immunomodulation.

Accordingly, a first object of the present invention relates to a method of treating an inflammatory skin disease in a subject in need thereof comprising administering to the subject a therapeutically effective amount of an urocanase inhibitor.

In a particular embodiment said method is associated with phototherapy.

A second object of the present invention relates to an urocanase inhibitor for use in a treatment of an inflammatory skin disease.

In a particular embodiment said use being associated with phototherapy.

A third object of the present invention relates to a Cosmetic composition comprising an effective amount of an urocanase inhibitor.

As used herein, the term "inflammatory skin disease" refers to diseases characterized by occurrence of a skin lesion resulting from infiltration of inflammatory cells such as activated helper T cells and monocytes. According to the present invention, inflammatory skin diseases comprise in particular contact dermatitis, atopic dermatitis, Netherton syndrome, SAM, SAMEC syndromes, psoriasis, polymorphic light eruption and vitiligo. As used herein the term "contact dermatitis" has its general meaning in the art and refers to a common inflammatory skin condition caused by exposure to exogenous substances. There are five primary types of CD: allergic CD (ACD), photoallergic CD (PACD), irritant CD (ICD), photoirritant CD (PICD) and protein CD (PCD). As used herein the term "atopic dermatitis" has its general meaning in the art and refers to a chronic disease affecting the skin. Atopic dermatitis is produced by a combination of genetic and environmental factors and associated with excessive IgE antibody formation. As used herein the term "Netherton syndrome" has its general meaning in the art and refers to a rare autosomal recessive genodermatosis caused by mutations in SPINK5 (LEKTI) one of the major inhibitor of the skin kallikrein cascade. As used herein the term "psoriasis" has its general meaning in the art and refers to a chronic inflammatory skin disease frequently associated with co morbidities, including arthritis, cardio-metabolic disease, and psychological troubles. It affects 2-3% of the world's population. Skin lesions present as erythematous scaly patches characterized by keratinocyte hyper-proliferation and inflammatory cell infiltrates in the epidermis and dermis. It is assumed that psoriasis results from abnormal dialogue between innate and adaptive immune cells in one hand and resident skin cells in another one. As used herein the term "**polymorphic light eruption**" has its general meaning in the art and refers to a common photodermatosis. It is typically characterized by nonscarring, pruritic, erythematous papules, plaques, or vesicles on sun-exposed skin that develop rapidly after sun exposure. As used herein the term "vitiligo" has its general meaning in the art and refers to a common chronic depigmenting disorder of the skin, with an estimated prevalence of 0.5-1% of the general population. Vitiligo is characterized by the development of white macules resulting from the loss of epidermal melanocytes. The pathophysiology is complex, combining genetic predispositions, environmental factors, metabolic alterations and dysregulation of the immune and inflammatory response.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein the term "urocanase" or "UROC1" (also known as "imidazolonepropionate hydrolase" or "urocanate hydratase") has its general meaning in the art and refers to the enzyme (EC 4.2.1.49 4.2.1.49) that catalyzes the second step in the degradation of histidine, the hydration of urocanate into imidazolonepropionate. Urocanase is encoded by the UROC1 gene in mammal (Gene ID: 131669). The protein itself is composed of 676 amino acids which then fold, producing the final product which has 2 identical subunits, making the enzyme a homodimer. To catalyze the hydrolysis of urocanate in the catabolic pathway of L-histidine the enzyme utilizes its two NAD+ (Nicotinamide Adnene Dinucleotide) groups. The NAD+ groups act as electrophiles, attaching to the top carbon of the urocanate which leads to sigmatropic rearrangement of the urocanate molecule. This rearrangement allows for the addition of a water molecule, converting the urocanate into 4,5-dihydro-4-oxo-5-imidazolepropanoate (Espinós C, et al (June 2009). Journal of Medical Genetics. 46 (6): 407-11)

Urocanase is found in some bacteria (gene hutU), in the liver of many vertebrates and has also been found in the plant Trifolium repens (white clover). Urocanase is a protein of about 60 Kd, it binds tightly to NAD+ and uses it as an electrophile cofactor (Retey J. 1994.Arch. Biochem. Biophys. 314:1-16.). Urocanase, is a homodimer with 557 amino acids in each subunit of the Pseudomonas enzyme (Kessler D, Retey J, Schulz GE. 2004. J. Mol. Biol. 342:183-194). The amino acid sequence is highly conserved (Espinos C, et al. 2009.. J. Med. Genet. 46:407- 411). For example, the urocanase of Pseudomonas shares about 35% identity with the human UROC1 gene product over the 500-amino-acid stretch corresponding to the bacterial enzyme.

Accordingly, in one embodiment, the methods of treatment of the present invention comprise a first step for determining whether the subject suffering from an inflammatory skin disease has Hut+ skin bacterial species (Proteobacteria and Staphylococcus spp.) selected from the list described in Table 1 and consisting of *Pseudomonas fluorescens, Pseudomonas fragi, Staphylococcus epidermidis, Pseudomonas azotoformans, Flavobacterium succinicans, Corynebacterium tuberculostearicum, Staphylococcus lentus Staphylococcus xylosus, Parabacteroides distasonis, Alistipes finegoldii, Staphylococcus caprae, etc.* Urocanase is widely distributed within the bacterial domain, though less so in the archaeal and eukaryotic domains. The Web resource Pfam (http: //pfam.sanger.ac.uk/) identifies 1,164 bacterial species with an ortholog of the urocanase gene. Of note, the amino acid sequence is also highly conserved. It shows more than 70% identity with the urocanase enzyme from plants, suggesting horizontal transfer (Bender, R.A. (2012). Regulation of the histidine utilization (hut) system in bacteria. Microbiol Mol Biol Rev 76, 565-584. 10.1128/MMBR.00014-12).

In some embodiments, the first step consists in detecting skin bacterial species expressing urocanase (Hut+ species). In some embodiments, the skin bacterial species is Staphylococcus epidermidis. One skilled in the art can easily identify such species using 16sRNA microbiome sequencing, and urocanase by western blot (as described in experimental data).

In skin bacterial species (Proteobacteria and Staphylococcus spp.) urocanase protein is encoded by the *HUTu* gene. One example of nucleotide sequence encoding bacterial urocanase is provided in SEQ ID NO:2 (ENA ref Sequence: D31856.1 Bacillus subtilis DNA, containing hut and wapA loci).

One example of wild-type bacterial Urocanase amino acid sequence is provided in SEQ ID NO:1 (Uniprot: P25503 · HUTU_ Bacillus subtilis (strain 168)):

MTDVKKSIRANRGTELECLGWEQEAVLRMLRNNLDPEVAEKPEDLIVYGGIG

KAARDWDAFHAIEHSLKTLKNDETLLVQSGKPVGMFRTHPQAPRVLLANSVLVPKW

ADWEHFHELEKKGLMMYGQMTAGSWIYIGSQGILQGTYETFAELARQHFGGSLKGT

LTLTAGLGGMGGAQPLSVTMNEGVVIAVEVDEKRIDKRIETKYCDRKTASIEEALAW

AEEAKLAGKPLSIALLGNAAEVHHTLLNRGVKIDIVTDQTSAHDPLIGYVPEGYSLDE

ADRLRQDTPELYVRLAKQSMKKHVEAMLAFQQKGSIVFDYGNNIRQVAKDEGLEN

AFDFPGFVPAYIRPLFCEGKGPFRWAALSGDPADIYRTDALLKELFPTNKALHRWIDM

AQEKVTFQGLPSRICWLGYGERKKMGLAINELVRTGELKAPVVIGRDHLDCGSVASP

NRETEAMKDGSDAVGDWAVLNALVNTAAGASWVSFHHGGGVGMGYSLHAGMVA

VADGSELADERLARVLTSDPGMGIIRHADAGYERAVEVAKEQDIIVPMQK

Of course, variant sequences of the urocanase may be used in the context of the present invention, those including but not limited to functional homologues, paralogues or orthologues of such sequences between the different bacterial species

## Urocanase inhibitors

As used herein the term "urocanase inhibitor" refers to a compound which is an inhibitor of the urocanase expression or urocanase activity. An "urocanase inhibitor" refers to a molecule (natural or synthetic) capable of neutralizing, blocking, inhibiting, abrogating, reducing or interfering with the biological activities of urocanase including, for example, reduction or blocking the interaction for instance between urocanase and cis-urocanic acid (cis-UCA) and/or inhibiting blocking urocanase enzymatic activity. Urocanase inhibitor include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Inhibitors also include, siRNA molecules directed to a protein, antisense molecules directed to a protein, aptamers, and ribozymes against a protein. For instance, the urocanase inhibitor may be a molecule that binds to urocanase and neutralizes, blocks, inhibits, abrogates, reduces or interferes with the biological activity of urocanase (urocanase enzymatic activity such as degradation/metabolization of cis-urocanic acid (cis-UCA)).
More particularly, the urocanase inhibitor according to the invention is:

1) an inhibitor of urocanase activity (such as small organic molecule, antibody, aptamer, polypeptide) and/ or
2) an inhibitor of urocanase gene expression (such as antisense oligonucleotide, nuclease, siRNA, ...)

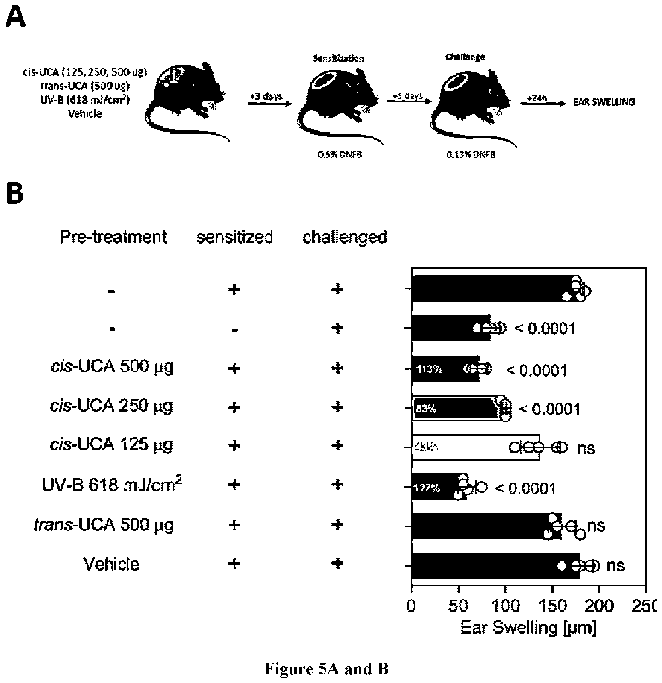

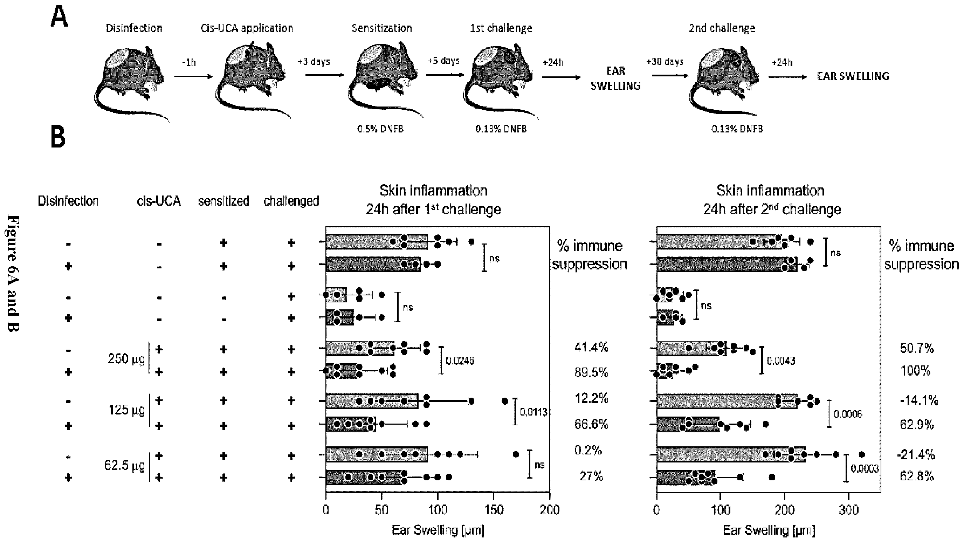

By "biological activity" of urocanase is meant in the context of the present invention, i) degradation/metabolization of cis-urocanic acid (cis-UCA ) and/or ii) regulation of the immunomodulatory properties of ultraviolet (UV) radiation.
Tests for determining the capacity of a compound to be a urocanase inhibitor are well known to the person skilled in the art. In a preferred embodiment, the inhibitor/antagonist specifically binds to urocanase (protein or nucleic sequence (DNA or mRNA)) in a sufficient manner to inhibit the biological activity of urocanase. Binding to urocanase and inhibition of the biological activity of urocanase may be determined by any competing assays well known in the art. For example, the assay may consist in determining the ability of the agent to be tested as a urocanase inhibitor/antagonist to bind to urocanase. The binding ability is reflected by the Kd measurement. The term "Kd", as used herein, is intended to refer to the dissociation constant, which is obtained from the ratio of Kd to Ka (i.e. Kd/Ka) and is expressed as a molar concentration (M). Kd values for binding biomolecules can be determined using methods well established in the art. In specific embodiments, an antagonist that "specifically binds to urocanase is intended to refer to an inhibitor that binds to urocanase polypeptide with a Kd of $1\mu M$ or less, 100nM or less, 10nM or less, or 3nM or less. Then a competitive assay may be settled to determine the ability of the agent to inhibit biological activity of urocanase. The functional assays based on UV induced immunomodulation procedures may be envisaged such as evaluating the ability to inhibit processes associated with cis-urocanic acid (cis-UCA ) metabolization mediated by urocanase biological activity (see example 1 and Figures 6 to 7).
The skilled in the art can easily determine whether a urocanase inhibitor/antagonist neutralizes, blocks, inhibits, abrogates, reduces or interferes with a biological activity of urocanase. To check whether the urocanase inhibitor/antagonist binds to urocanase and/or is able to restore processes associated with UV induced immunomodulation (for instance, through restoration of cis-UCA) activity) in the same way than the initially characterized inhibitor of urocanase, binding assay and/or a cis-UCA activity assay may be performed with each antagonist. For instance, restoration of cis-UCA activity can be assessed by detecting active cis-UCA with HPLC-MS, and/or by assessment of enzymatic activity of urocanase on cis-UCA formation in presence of trans-UCA in biological samples. Restoration of cis-UCA activity can also be assessed by detecting cis-UCA-induced immunomodulation in an allergy model (see Figures 5 and 6) (see also references (17) and (18)) or in standard *in vitro* T cell suppression or dendritic cell maturation assays (Vladimir Holáň; Lucia Kuffová; Alena Zajícová; Magdaléna Krulová; Martin Filipec; Petr Holler; Alexander Jančárek. Urocanic Acid Enhances IL-10 Production in Activated CD4+ T Cells. J Immunol (1998) 161 (7): 3237-3241 ; F M Rattis 1 , J Péguet-Navarro, P Courtellemont, G Redziniak, D Schmitt. Cis-urocanic acid failed to affect in vitro human Langerhans cell allostimulatory function. Photochem Photobiol. 1995 Nov;62(5):914-6. doi: 10.1111/j.1751-1097.1995.tb09155.x).

Accordingly, the urocanase inhibitor/antagonist may be a molecule that binds to urocanase selected from the group consisting of small organic molecules antibodies, aptamers, and polypeptides.

The skilled in the art can easily determine whether a urocanase inhibitor/antagonist neutralizes, blocks, inhibits, abrogates, reduces or interferes with a biological activity of urocanase : (i) binding to urocanase (protein or nucleic sequence (DNA or mRNA)) and/or (ii), restore cis-UCA activity (UV induced immunomodulation) through blocking the urocanase activity or expression.

Accordingly, in a specific embodiment the urocanase inhibitor/antagonist directly binds to urocanase (protein or nucleic sequence (DNA or mRNA)) and allows to restore cis-UCA activity to mediate UV induced immunomodulation.

Thus in a second aspect the present invention also relates to a urocanase inhibitor/antagonist for use in a method to restore the cis-UCA/activity of a patient affected with an inflammatory skin disease.

Inhibitors of the urocanase expression

A first object of the present invention relates to a urocanase inhibitor which is an inhibitor of the urocanase expression for use in the treatment of an inflammatory skin disease.

By "inhibitor of the urocanase expression", it is herein referred to a compound which is capable of reducing or suppressing the synthesis of functional urocanase.

Inhibitors of urocanase gene expression for use in the present invention may be based on antisense oligonucleotide constructs. Anti-sense oligonucleotides, including anti-sense RNA molecules and anti-sense DNA molecules, would act to directly block the translation of urocanase mRNA by binding thereto and thus preventing protein translation or increasing mRNA degradation, thus decreasing the level of urocanase, and thus activity, in a cell. For example, antisense oligonucleotides of at least about 15 bases and complementary to unique regions of the mRNA transcript sequence encoding urocanase can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of urocanase gene expression for use in the present invention. Urocanase gene expression can be reduced by contacting a subject or cell with a small double stranded RNA (dsRNA), or a vector or construct causing the production of a small double stranded RNA, such that urocanase gene expression is specifically inhibited (i.e. RNA interference or RNAi). Methods for selecting an appropriate dsRNA or dsRNA-encoding vector are well known in the art for genes whose sequence is known (e.g. see for example Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Examples of commercial siRNAs against urocanase include but are not limited to: UROC1-set siRNA/shRNA/RNAi Lentivector Cat. No.49251091 Abeam; urocanase domain containing 1 siRNA ID MSS216781 Catalog #1320001, Thermofisher; UROC1 siRNA (h) catalogue : sc-78038 Santa Cruz.

Ribozymes can also function as inhibitors of urocanase gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of urocanase mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of urocanase gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing urocanase. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUCl9, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, eye, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In a preferred embodiment, the antisense oligonucleotide, siRNA, shRNA or ribozyme nucleic acid sequence is under the control of a heterologous regulatory region, e.g., a heterologous promoter.

Inhibitors of the urocanase activity

A second aspect of the present invention relates to a urocanase inhibitor/antagonist which is an inhibitor of the urocanase activity for use in the treatment of skin inflammatory disease.

By "inhibitor of the urocanase activity", it is herein referred to a compound which is capable of restore or inducing cis-UCA activity to mediate UV induced immunomodulation (see above). In view of the teaching of the present disclosure, particularly of the examples, it falls within the ability of the skilled person to assess whether a compound is an inhibitor of the urocanase activity. For example, a suitable test consists in evaluating if said compound restore cis-UCA activity to mediate UV induced immunomodulation. A suitable test for detecting cis-UCA induced UV induced immunomodulation is described in examples hereinafter.

In a particular embodiment, the present invention relates to a compound which is an inhibitor of the urocanase activity for use in the treatment of inflammatory skin diseases, wherein said compound is an anti-urocanase antibody which neutralizes urocanase activity or an anti-urocanase antibody fragment which neutralizes urocanase.

Antibodies directed against urocanase can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies against urocanase can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see e.g., U.S. Pat. No. 4,946,778) can be adapted to produce anti-urocanase single chain antibodies. Urocanase activity inhibitors useful in practicing the present invention also include anti-urocanase antibody fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to urocanase.

Humanized anti-urocanase antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

Then, for this invention, neutralizing antibodies of urocanase are selected.

In still another embodiment, urocanase activity inhibitors may be selected from aptamers. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of a conformationally constrained antibody variable region displayed by a platform protein, such as E. coli Thioredoxin A that are selected from combinatorial libraries by two hybrid methods (Colas et al., 1996).

Then, for this invention, neutralizing aptamers of urocanase are selected.

In a particular embodiment, the compound according to the invention is an anti-urocanase polypeptide like glycineglycine.

Glycylglycine is the dipeptide of glycine. The compound was first synthesized by Emil Fischer and Ernest Fourneau in 1901 by boiling 2,5-diketopiperazine (glycine anhydride) with hydrochloric acid (R.H.A. Plimmer (July 2008) [1908]. R.H.A. Plimmer & F.G. Hopkins (ed.). Vol. Part II (1st ed.) Shaking with alkaliand other synthesis methods have been reported (Dunn, Max S.; et al (1932). Journal of Biological Chemistry. American Society for Biochemistry and Molecular Biology. 99 (1): 217-220). Because of its low toxicity, it is useful as a buffer for biological systems with effective ranges between pH 2.5-3.8 and 7.5-8.9; however, it is only moderately stable for storage once dissolved (Smith, Marshall E.; et al (1949). ".The Biological Bulletin. Woods Hole, MA: Marine Biological Laboratory. 96 (3): 233-237). Glycylglycine was also described as a specific inhibitor of bacterial and bovine urocanases (Hunter JK et al Pept Res. 1989 May-Jun;2(3):240-5).

In dermo-cosmetology Glycylglycine is used as antiaging agent for protecting the support fibers of the connective tissue against glycation in the form Glycylglycine oleamide (see WO1999048470) .

The glycylglycine oleamid is a lipo-peptide or acyl-peptide resulting from the condensation of oleic acid and glycylglycine dipeptide and can also be us in the method of the present invention.

By a "therapeutically effective amount" of the active agent as above described is meant a sufficient amount to provide a therapeutic effect. It will be understood, however, that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the invention, the active agent is administered to the subject in the form of a pharmaceutical composition. Typically, the active agent may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and fluid, as long as it can be easily syringed. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The active agent can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumour area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

In a preferred embodiment the active agent (uroccanase inhibitor) is administrated topically on the skin.

The term "phototherapy", also called "Light therapy" or "bright light therapy" has its general meaning in the art and refers to the exposure to direct sunlight or artificial light at controlled wavelengths in order to treat a variety of medical disorders, including seasonal affective disorder (SAD), circadian rhythm sleep-wake disorders, cancers, and skin wound infections. Treating skin conditions such as neurodermatitis, psoriasis, acne vulgaris, and eczema with ultraviolet light is called ultraviolet light therapy.

Thus, the phototherapy of the present invention is preferably performed by ultraviolet light therapy. The Ultraviolet light therapy or ultraviolet phototherapy is a typically the treatment for psoriasis, atopic skin disorder, vitiligo and other skin diseases and there are two main treatments: UVB (Wavelength 280-315 nm) that is the most common, and PUVA.

Ultraviolet light therapy at the target site (skin) with LED lamps, lasers or other light sources via fluorescent bulb or any other appropriate method knows in the art.

PUVA means psoralen and UVA (Wavelength 280-315 nm). It consists of irradiation of the skin with the UVA ultraviolet light, from a fluorescent bulb or LED lamps and is an ultraviolet light therapy treatment for skin diseases: vitiligo, eczema, psoriasis, graft-versus-host disease, mycosis fungoides, large plaque parapsoriasis, and cutaneous T-cell lymphoma, using the sensitizing effects of the drug psoralen. The psoralen is applied or taken orally to sensitize the skin, then the skin is exposed to UVA.

There are four UVB types of lamps: Fluorescnt Broad-Band UVB that emit 280-330 nanometer, Fluorescent Narrow-Band that emit 312 nanometer, Excimer that emit 308 nanometer and LED that emit 290-300 nanometer. Tanning beds are used both in dermatology practices for the treatment of cosmetic skin conditions (such as psoriasis, acne, eczema and vitiligo) and in indoor tanning salons for cosmetic tanning.

Typical treatment regimens involve short exposure to UVB rays 3 to 5 times a week at a hospital or clinic, and repeated sessions may be required before results are noticeable. Almost all of the conditions that respond to UVB light are chronic problems, so continuous treatment is required.

The present invention provides mechanistic evidence that certain cutaneous bacterial species can metabolize the UVB mediator, cis-UCA, thus reducing its concentration, and thereby diminishing UV-induced immunomodulation.

Accordingly in preferred embodiment the phototherapy associated with the urocanase inhibitor is UVB phototherapy.

In a particular embodiment the urocanase inhibitor is administered prior to phototherapy.

The invention will be further illustrated by the following figures and examples. However, these examples and figures should not be interpreted in any way as limiting the scope of the present invention.

**FIGURE LEGENDS**

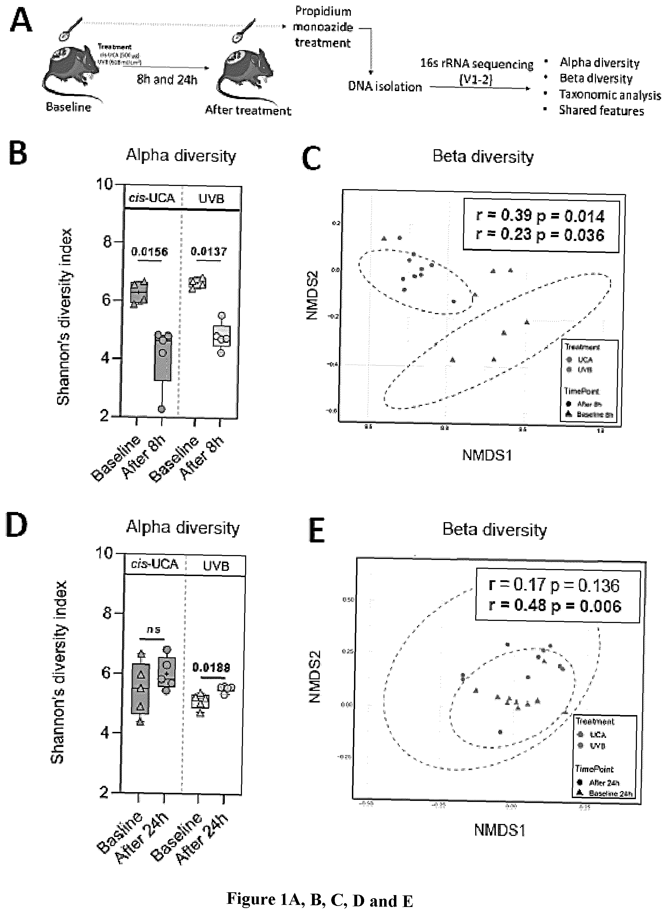

**Figure 1: Transient changes in skin bacterial diversity after topical administration of cis UCA or UVB exposure.** C57BL/6 mice were exposed on shaved dorsal skin to 500 $\mu$g cis-UCA or 618 mJ/cm2 UVB and bacterial 16S rRNA amplicon sequencing (regions V1-V2) was performed on skin swabs collected at baseline and 8 or 24 hours after treatment. Alpha diversity and beta diversity metrics were calculated in each sample (A). Shown are box-and-whisker plots with individual data of Shannon diversity index (alpha diversity) and non-metric multidimensional scaling (NMDS) order (beta diversity) at 8h (B&C) and 24h (D&E) versus baseline. Results are representative of two independent experiments with 5 mice per group. P values were determined by paired t-test (B&D) and analysis of variance (ADONIS) calculation for group differencesbased on Bray-Curtis distance (C&E).

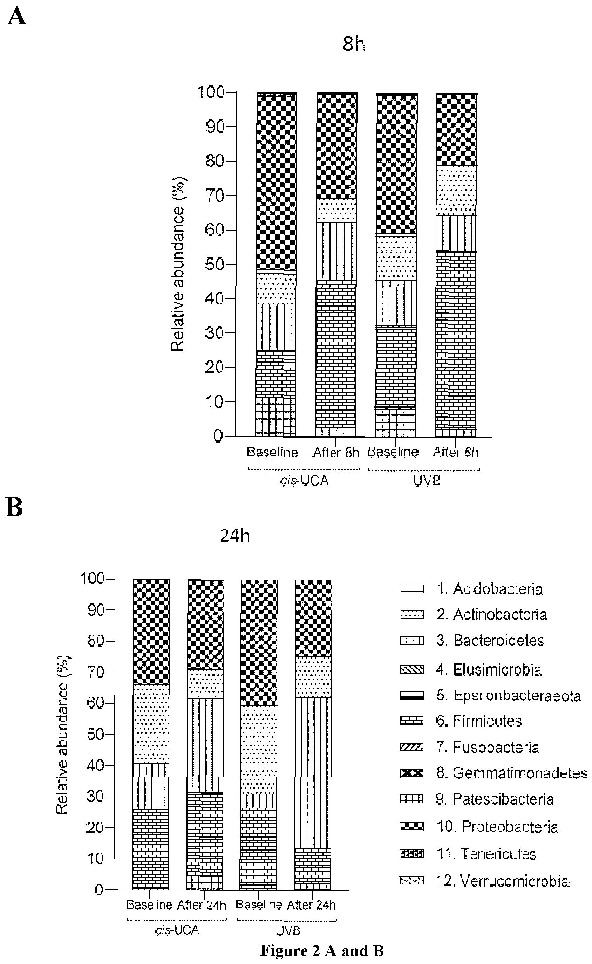

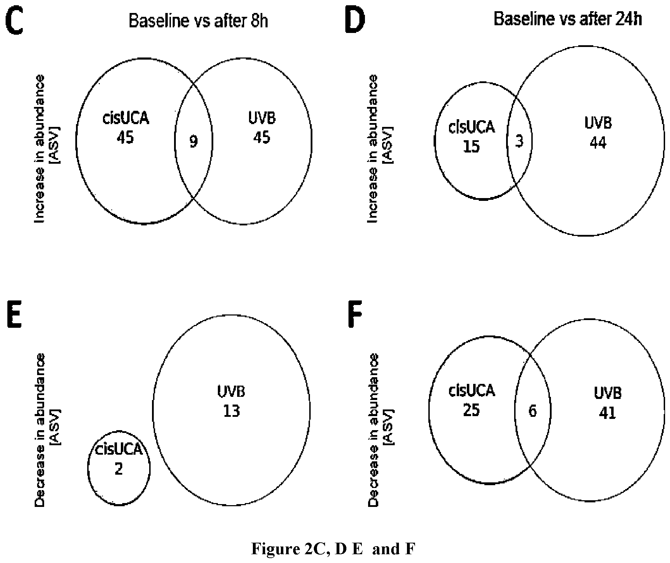

**Figure 2: Changes in skin phylum and species composition after topical administration of cis-UCA or UVB exposure.** C57BL/6 mice were exposed to cis-UCA or UVB, and the nature of amplicon sequencing variants (ASVs) in each swab was characterized as in Figure 1. Data depict the mean relative abundance of major bacteria phyla (colored bar stacks) at 8h (A) and 24h (B) versus baseline, and the number of species with increased (C&D) or decreased (E&F) abundance after respective treatment, including those common to the two treatments.

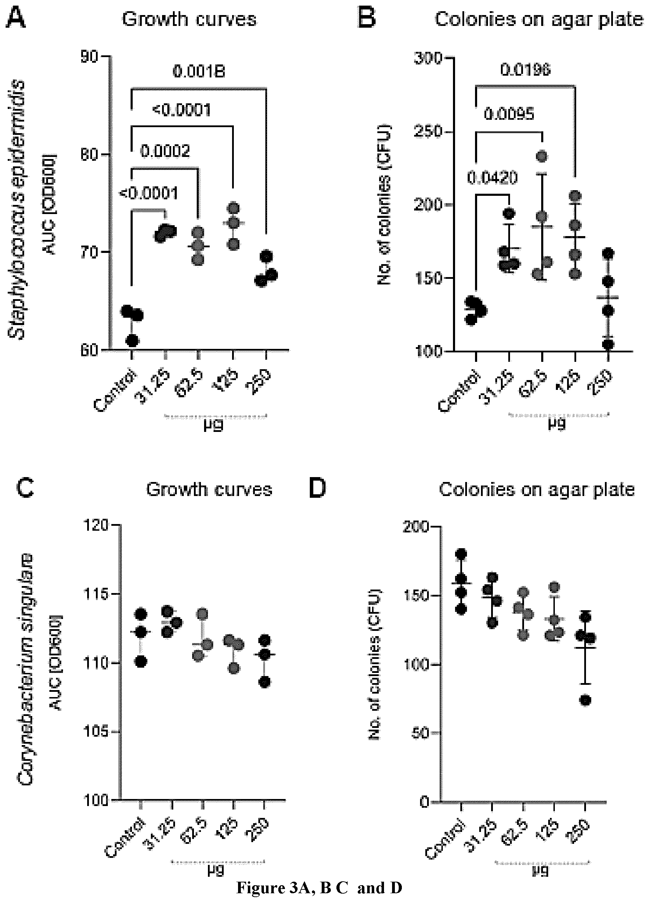

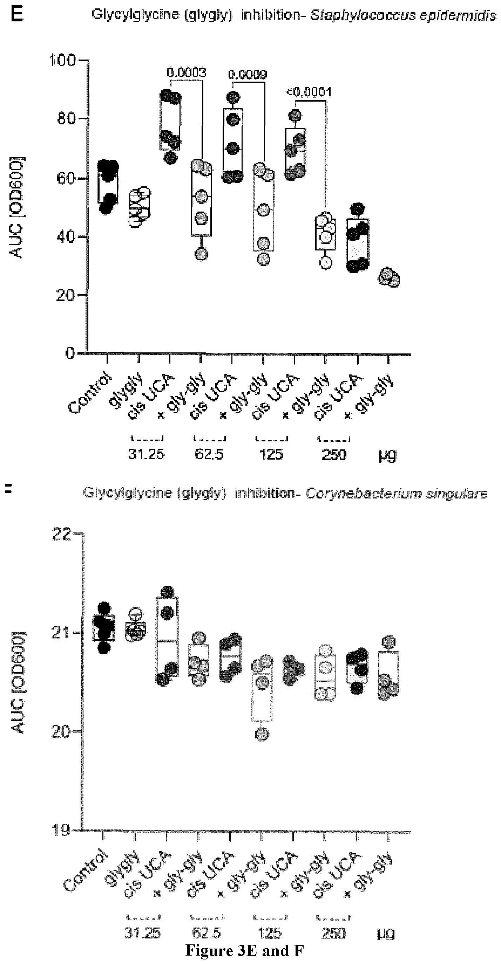

**Figure 3: Certain bacterial species utilize cis-UCA for growth.** Staphylococcus epidermidis (A, B, E) and Corynebacterium singulare (C, D, F) strains were cultured in liquid Luria Broth medium (for 24 hours) or on solid agar plates (for 48 hours), in the presence of different concentrations of cis-UCA (from 31.25 to 250 $\mu$g). Bacteria growth was measured by spectrophotometry or by counting the number of colony forming units (CFUs). Results depict means $\pm$ SD and individual data of the areas under the curve (AUC), calculated and plotted from OD (600nm) values obtained from 24h-growth curves (A, C), or of the number of CFUs (B, D). In some experiments, glycylglycine (from 31.25 to 250 $\mu$g) was added in Luria Broth medium to inhibit UCA catabolism (E-F). Data are representative of two independent experiments. P values were determined using the 2-way ANOVA.

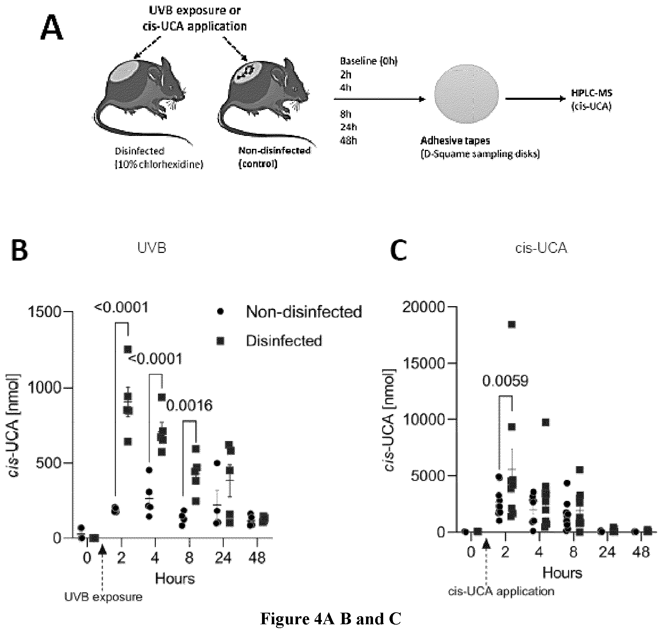

**Figure 4: cis-UCA persists on the skin depending on the microbiome.** C57BL/6 mice, disinfected onto the skin with chlorhexidine or left untreated, were exposed 1 hour later to cis- UCA or UVB as in Figure 1. Skin samples were collected at different time points (from baseline to 48 hours) using adhesive tapes, and the presence of cis-UCA was analyzed by HPLC-MS (A). Results depict means $\pm$ SEM and individual quantitative data after topical application of cis-UCA (B) or UVB irradiation (C). Data are pooled from two independent experiments with 5-10 mice per group. P values were determined using Two-way ANOVA

**Figure 5: Cis-UCA induces dose-dependent systemic immunosuppression against DNFB.** C57BL/6 mice were pretreated on shaved dorsal skin with graded doses (125, 250, and 500 ug) of cis-UCA, with trans-UCA (500ug), UVB (618 mJ/cm2), vehicle (70% EtOH) or left untreated. Three days later, mice were sensitized with 0.5% DNFB (25ul) on the shaved abdomen (except those from the "irritation control" group), and next challenged onto the ears with 0.13% DNFB (2x5ul) 5 days later. Results depict means $\pm$ SD and individual ear swelling data, as measured 24 hours after DNFB challenge. The percentage of immunosuppression induced in cis-UCA-versus vehicle-treated groups is also shown. Data are pooled of two different experiments with 5 mice per group. P values were determined using Dunnett's test for multiple comparisons. ns,not significant.

**Figure 6. The skin microbiome limits cis-UCA mediated immunomodulation and tolerance to DNFB.** C57BL/6 mice, disinfected with chlorhexidineor left untreated, were treated 1 hour later with graded doses of cis-UCA (62.5, 125 and 250ug) or with 70% EtOH vehicle. Animals were next sensitized and challenged with DNFB, as in Figure 5. A second DNFB challenge was also performed on the same ear 30 days later to assess the local memory response (A). Results depict means $\pm$ SD and individual ear swelling data, as measured 24 hours after the first and the second DNFB challenge. The percentage of cis-UCA induced immunomodulation in chlorhexidine-treated groups and non-disinfected controls is also shown (B). Data are pooled from two independent experiments with 2-4 mice per group. P values were determined using the Mann-Whitney test.

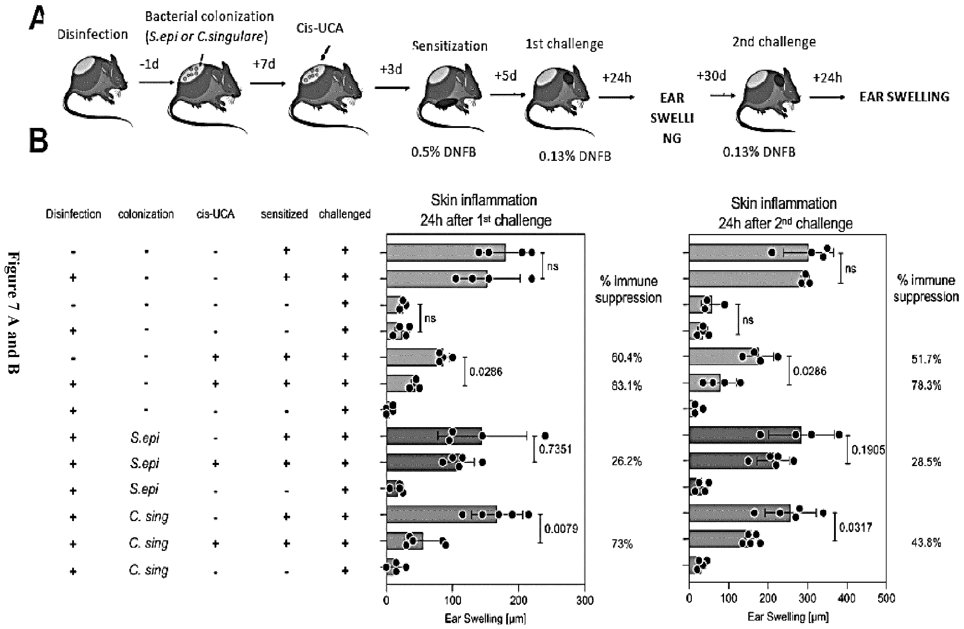

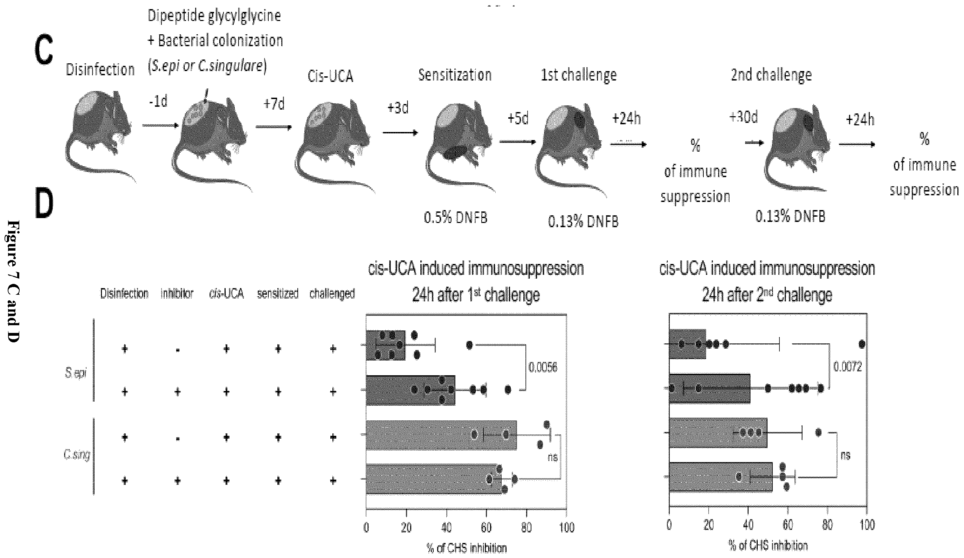

**Figure 7. Urocanase positive bacteria limit cis-UCA-induced immunomodulation.** C57BL/6 mice, disinfected or not with chlorhexidine, were colonized 1 day later with either 109 CFU of S. epidermidis (red bars) or C. singulare (green bars), or left untreated (grey bars). After 7 days, mice were treated with 125ug cis-UCA or with 70% EtOH vehicle and next sensitized and challenged with DNFB, as in Figure 5. A second DNFB challenge was also performed on the same ear 30 days later to assess the local memory response (A). Results depict means $\pm$ SD and individual ear swelling data, as measured 24 hours after the first and the second DNFB challenge. The percentage of cis-UCA-induced immunomodulation in recolonized and control groups is also shown (B). In some experiments, mice were treated with dipeptide glycylglycine (250ug) at the time of bacterial colonization (C), and the percentage of cis-UCA-induced immunosuppression versus controls, which did not receive the urocanase inhibitor, was calculated. Results depict means $\pm$ SD and individual data, as measured 24 hours after the first and the second DNFB challenge (D). Data are representative (B) or pooled (D) from two independent experiments with 4-5 mice per group. P values were determined using the Mann-Whitney test (B) or the Two-way ANOVA (D).

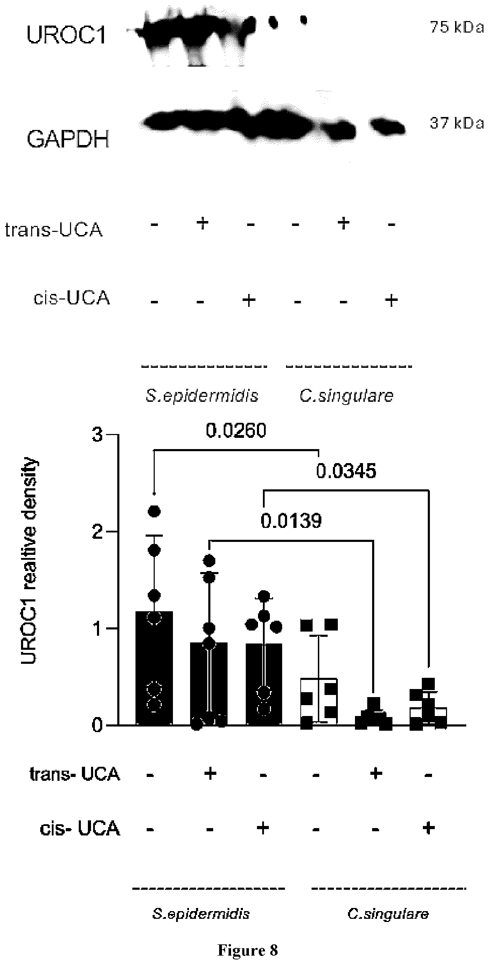

**Figure 8. *Staphylococcus epidermidis* but not *Corynebacterium singulare* expresses urocanase.** *Staphylococcus epidermidis* and *Corynebacterium singulare* strains were cultured in liquid Luria Broth medium for 24 h. In some cultures, 250 $\mu$g *cis-UCA* or 250 $\mu$g trans-UCA were added. At the end of the culture experiment the presence for urocanase (UROC1) or control GAPDH protein was quantified by western blot. Results depict representative immunoblots (left side) and means ± SD and individual protein expression data in 24 h-cultures. P-values were determined using the two-way ANOVA.

**EXAMPLE:**

**Methods:**

**Experimental procedures (STAR methods)**

**Data and Code Availability**

Data were uploaded to ENA (European Nucleotide Archive), supported by EMBL-EBI, and assigned the primary accession number PRJEB65006. This paper does not report any original code. Any additional information required to reanalyze the data reported in this paper is available from the lead author upon request.

**Experimental model and subject details**

**Chemicals**

Cis-UCA (#U6883) was purchased from Sigma-Aldrich (Saint-Quentin Fallavier, France), trans-UCA (#T37547 from TargetMol (Massachusetts, United States) and these were dissolved in ethanol/milliQ water (EtOH; 7:3 vol/vol). 2,4-dinitrofluorobenzene was purchased from Sigma-Aldrich (Saint-Quentin Fallavier, France) and dissolved in an acetone/olive oil (AOO) vehicle (4:1 vol/vol) for epicutaneous application. Chlorhexidine (#282227) was purchased from Sigma-Aldrich (Saint-Quentin Fallavier, France) and was dissolved in isopropyl alcohol. Dipeptide glycylglycine (#PHR2733) was purchased from Sigma-Aldrich (Saint-Quentin Fallavier, France) and was dissolved in sterile water.

**Bacteria**

We performed species level identification from the 16s data using the EzBioCloud database, the analysis assigned species level taxonomy to specific strains, these strains were used for in vitro experiments. The Staphylococcus epidermidis strain was obtained from ATCC (ATCC14990; LGC standards, France) and Corynebacterium singulare from the Leibniz Institute DSMZ (DSMZ44357; Braunschweig, Germany).

**Animals**

Female C57BL/6J mice were purchased from Charles-River Laboratories (Sulzfeld, Germany or Saint Germain Nuelles, France). Mice were used between 6 and 10 weeks of age and were housed in groups in autoclaved cages. Sterile food and water were provided ad libitum. One important factor to be considered during the experimental design for microbiome studies (30) is the transfer of the fecal microbiome to the skin that occurs in the animals' cages. To avoid this, the cages were changed every day during each experiment, and known fecal microbes were removed during data analysis. To prevent any cage effect (31), all mice were grouped together once per week and then mice were randomly housed in cages.

**Study approval**

All animal care and treatment protocols were approved by the Austrian Federal Ministry for Education, Science and Research (protocol number BMWFW-66.010/0137-WF/V/3b/2014). Some experiments were conducted at the Plateau de Biologie Expérimentale de la Souris (PBES, SFR Biosciences Gerland-Lyon Sud UMS344/US8, France) under specific pathogen-free conditions. Protocols were also approved by the local institutional review board (Comite Régional d'Ethique sur l'Expérimentation Animale) and the French authorities (approval no: APAFIS#31304-2021021214481404).

The clinical study protocol was approved by the local ethics committee at the Medical University of Graz, Austria, and the study (application no: 33-196 ex 20/21) was executed in accordance with the principles of the Declaration of Helsinki. All study procedures were performed between April 2021 and May 2021 at the Photodermatology Research Unit, Medical University of Graz, Austria. Each eligible participant signed a written informed consent before study entry.

**Detailed methods**

**UCA treatment**

The backs of the mice were shaved using an electric clipper 24 h prior to UCA applications or UV-B exposure. Mice were applied with 100 μl of cis- or trans-UCA to deliver from 125 to 500μg of compound to the shaved dorsal skin on the indicated days. For the microbiome analysis experiment cis-UCA was dissolved in water, and in 70% ethanol for CHS experiments.

**UV irradiation in mice**

UV radiation was performed using a Waldmann 236 light source (Waldmann Medizintechnik, Villingen-Schwenningen, Germany) equipped with two Waldmann UV6 fluorescent tubes (emission range 280-370 nm; peaking in the UVB range at 313 nm; Commission Internationale de l'Éclairage (CIE)-erythema-weighted peak at 300nm) and positioned upside down on top of cage. The mean UVB irradiance of the lamp was 1.9 mW/cm2 as measured by a Waldmann UV photometer with a UV6 detector head appropriated for the radiation device.

The shaved dorsal skin of the mice was exposed to UVB (618 mJ/cm2) over an average time of exposure of 5 min 42 sec. In CHS experiments, the ears were protected from UVB exposure by covering them with black electrical tape. All the procedures were performed in a laminar air flow unit.

**UV irradiation in human volunteers**

Skin samples from 6 healthy control individuals were available from a pilot study (ClinicalTrials.gov identifier: NCT04985526). Briefly, the effects of the skin disinfectant Octeniderm® (Schuelke & Mayr GmbH, Austria), made of 1-propanol, 2-propanol, and octenidine dihydrochloride, was used in this study, and these effects were compared to those of conventional sodium chloride (0.9%, Fresenius Kabi GmBH, Austria) as a control. The treatments were applied to the test fields 24 h before solar-simulated ultraviolet radiation (SSUVR) exposure and again immediately (within 10 min) before exposure. Twelve mL of the commercial disinfectant or sodium chloride were applied to the skin. The Waldmann UV 801 KL system (Waldmann GmbH, Villingen-Schwenningen, Germany) equipped with helium lamps (Helarium Wolff Systems Arimed B40 light tubes, B1-12-40W/BIPIN) was used in this study. The mean irradiance (290-400 nm) at the skin level (at a distance of 15 cm from the outermost light source filter) was 2.00 mW/cm2. The physical doses were increased stepwise, ranging from 0.71 J/cm2 (test field 1) to 4.00 J/cm2. The irradiation time necessary to administer these doses ranged from 5min 53s to 33min 20s.

**Disinfection procedure**

Mice were disinfected using freshly prepared 10% chlorhexidine in 70% isopropyl alcohol solutions. Wipes were soaked in solution and subsequently rubbed gently for at least 30s each on the dorsal skin of the mice, at 24 h and 1 h before the UCA application or UVB irradiation.

To check for disinfection efficacy, skin swabs were collected at different time intervals (from baseline to 14 days) from the shaved, disinfected backs by gently rubbing skin with swabs for 30s. Each swab was then soaked in 100 μL of tryptic soy broth (#BA-257107.06, Becton Dickinson GmbH, Germany) medium and was plated on solid agar. The numbers of colony-forming units were counted after 24 h of culture at 37°C.

**Bacterial cultures and growth assays**

Staphylococcus epidermidis ATCC14990 and Corynebacterium singulare DSM44357 were revived on Luria agar and cultured in liquid Luria Broth (LB) medium at 37 °C with shaking at 200-250 rpm. Optical density measurements for bacterial cultures were obtained using a Tecan M200 spectrophotometer and a Costar flat-bottomed 96-well plate with a lid and adding 200 μL per well for all measurements. Bacteria were cultured for 24 h in LB, and dilutions for measurement were made in the same medium. The absorbance was measured at a wavelength of 600 nm at 37 °C, and the mean of 4 readings per well was taken and plotted in GraphPad Prism software to visualize the growth curves from zero to 24h. Additionally, the area under curve from the growth curves (OD600 readings from zero to 24h) were calculated and plotted. Bacteria were also cultured on solid agar plates by applying 100 μL of suspended cultures to the plate and streaking these gently. The agar plates were incubated at 37 °C, and the numbers of number of colony-forming units were counted after 48 h. In some experiments, 20 μL of dipeptide glycylglycine (containing 31.25-250 μg of compound) were added to the medium at the start of the culture.

**Generation of gnobiotic-like models**

To generate gnobiotic-like models, C57BL/6 mice that had been previously shaved and disinfected with chlorhexidine and isopropyl alcohol on their dorsal skin were gently rubbed with $1 \times 109$ CFU/mL (~100 $\mu$l) of S. epidermidis or C. singulare cultures by using sterile cotton swabs soaked in the bacterial cultures, as described previously.(32, 33) This bacterial application was repeated every other day on four occasions. In some groups, dipeptide glycylglycine (250 $\mu$g) was added in bacteria culture (1 mL) to block the catabolism of UCA, and then applied (~100 $\mu$l/ mice) together with the bacteria to shaved mouse skin.

**Contact hypersensitivity (CHS) assay**

The CHS assay was performed as previously described.(5) In brief, mice were sensitized epicutaneously on their shaved abdomen with 25 $\mu$L of 0.5% DNFB 3 days after UCA application or UVB irradiation. Five days later, they were challenged on the left ear with 10 $\mu$L (5 $\mu$L on each side of the ear) of 0.13% DNFB, while the right ear received the acetone/olive oil vehicle. Flare-up reactions in some allergic animals were also induced by re-applying 10 $\mu$L of 0.13% DNFB (5 $\mu$L to each side of the ear) to healed lesions 30 days later.

Ear thickness was measured by using a spring-loaded micrometer (J15, Blet SA, Lyon, France) before and 24 h after the first or second challenge on both ears, and then mean values were calculated. Ear swelling was calculated by subtracting the initial value from the value recorded on the corresponding day and by further subtracting any swelling recorded for the vehicle-control ear from the swelling recorded for the hapten-challenged ear.

The percentage of UCA- or UVB-induced immunosuppression was also calculated by using the following formula.

$$\% \; immune \; suppression = \left[ 1 - \left( \frac{A-B}{C-B} \right) * 100 \right]$$

**A:** Ear swelling of animals sensitized and challenged with DNFB 3 days after UCA or UVB pre-treatment
**B:** Ear swelling of animals challenged with DNFB only (not sensitized) (negative control group)
**C:** Ear swelling of animals sensitized and challenged with DNFB but not pre-treated with UCA or UVB (positive control group)

**Microbiome sampling**

Skin swabs were collected from the shaved backs of the mice using the BD CultureSwab™ EZ Collection and Transport System (#220144, Becton Dickinson GmbH, Germany) soaked with sterilized 0.15M NaCl in 0.1% Tween-20 buffer.(34) The tips of the swabs were then cut under sterile conditions and placed into sterile 1.5-mL Eppendorf tubes containing 500 $\mu$L of buffer (NaCl + Tween-20). To remove or exclude dead or damaged bacteria, PMA™ (propidium monoazide) (#40013 from Biotium, California, USA) was added to each reaction tube (0.87 $\mu$L of 20 mM) and left at room temperature for 5 min in the dark, then subjected to photoactivation for 15 min using a PMA-Lite™ LED Photolysis Device (Biotium, #E90002).(35) Unused swabs soaked in sterile buffer (0.15 M NaCl in 0.1% Tween-20) were used as negative controls. The samples were then frozen at -80 °C immediately until further processing.

**Microbial DNA isolation**

Total DNA was isolated from the frozen skin swabs by using both mechanical and enzymatic lysis. The samples were thawed and transferred to a sterile Magna Lyser green bead tube (Roche, Manheim, Germany). These were subjected to mechanical lysis at 6000 rpm for 30 s twice in a MagNA Lyser Instrument (Roche, Mannheim, Germany). Unused swabs and unused buffer tubes without swabs served as negative controls for sampling and DNA isolation. The samples were further subjected to enzymatic lysis by incubating them with 2.5 $\mu$l of lysozyme (100 mg/mL; Carl Roth, Karlsruhe, Germany) at 37 °C for 1 h. Further processing was performed using QIAamp DNA microbiome kit (#51704, Qiagen, Hilden, Germany) per the manufacturer's instructions. Total DNA was eluted in the 35 $\mu$l of elution buffer provided in the kit and stored at -20 °C until PCR amplification. All procedures were performed under sterile conditions.

**16S microbiome sequencing**

16s rRNA library preparation, quantitation, and sequencing were performed as described previously (34, 36). Briefly, 5 $\mu$L of total DNA were used for PCR amplification with the 16s rRNA target specific primers 27F 5'-AGAGTT

TGATCCTGGCTCAG-3' (SEQ ID N°3) and R357 5'- CTGCTGCCTYCCGTA-3' (SEQ ID N°4) (MWG, Ebersberg, Germany). The PCR reaction started with denaturation at 95 °C for 3 min followed by 32 cycles of denaturation at 95 °C for 45 s, annealing at 55 °C for 45 s and extension at 72 °C for one min. Final extension was performed at 72°C for seven min. The PCR reactions were performed in triplicates, which were then pooled and 5 $\mu$L visualized on a 1% agarose gel for amplification success. Normalization, indexing, and pooling of the individual PCR products and purification of the final library were performed as previously described.(34, 36) The library was quantified using a QuantiFluor ONE dsDNA Kit (Promega, Mannheim, Germany) according to manufacturer's instructions and visualized on an Agilent 2100 Bioanalyzer (Agilent Technologies, Waldbronn, Germany) using a high-sensitivity DNA assay according to the manufacturer's instructions. The 6-pM (picomolar) library was sequenced on a MiSeqII desktop sequencer (Illumina, Eindhoven, Netherlands) with v3 chemistry for 600 cycles; 20% PhiX control DNA and FastQ files were used for data analysis.

**16s bioinformatic analysis**

Illumina paired-end raw sequencing data were first checked for quality and the presence of sequencing adapters. Quantitative Insights into Microbial Ecology (QIIME2 Version 2019.7),(37) a bioinformatic pipeline integrated in the open-source web-based platform Galaxy (https://galaxyproject.org/) hosted on the MedBioNode HPC cluster at the Medical University Graz, Austria (https://galaxy.medunigraz.at/), was used to analyse the final sequence files. A total of 2,669,038 (66,725 $\pm$ 22,376) raw sequence reads were quality-filtered, de-noised, de-replicated, merged, and checked for chimeras using the DADA2 denoise pipeline(38) with optimized parameters: p-trunc-len-f: 220, p-trunc-len-r: 200, p-trim-left-f: 20, p-trim-left-f: 15, and p-max-ee: 2.0 as implemented in the QIIME2 microbiome bioinformatics platform. Taxonomic assignment of the DADA2 representative sequence set (ASV) was accomplished with the QIIME2 sklearn-based classifier against the SILVA rRNA database (Release 132) at 99% identity.(39) All further downstream statistical data analyses including alpha and beta diversity analyses were conducted with the R version 4.1.0 program for statistical computing (https://www.R-project.org) and the vegan, ggplot2, and psych packages.

**HPLC-MS**

Tape strips (#D100, D-Squame, Texas, USA) or bacterial culture supernatant were submersed or mixed in 1 mL of methanol and shaken for 10 min on a shaker at 800 rpm. Subsequently 800 $\mu$L of the supernatant were transferred into an autosampler vial, evaporated in a SpeedVac at room temperature and resuspended in 200 $\mu$L of 0.5% formic acid. Five $\mu$L were injected into an UHPLC-MS system (Ultimate 3000 and TSQ Quantum Ultra, both Thermo Fisher Scientific) at a flow rate of 150 $\mu$L/min equipped with a 150 x 2.1 mm, 2.6 $\mu$m Kinetex C18 EVO HPLC column (Phenomenex, California, USA). The isocratic eluent was aqua bidest. containing 0.5% formic acid and 5 mM ammonia formate. The TSQ triple quadrupole mass spectrometer acquired the mass transitions m/z 139.1/83.1 and m/z 139.1/121.1, both at a 10-eV collision energy using argon as the collision gas for cis-UCA and trans-UCA alike. Since these metabolites are cis/trans isomers, they can be separated by retention time but not by mass/fragment sizes. Quantification was performed by running an external calibration curve from 1.1 nM to 6 $\mu$M for both trans-UCA and cis-UCA. Data were uploaded to European Nucleotide Archive, supported by EMBL-EBI, and assigned the primary accession number PRJEB74912.

**Immunoblot**

Frozen samples were lysed in a lysis buffer (0.05 M Tris-HCl, 0.15 mM NaCl, 0.5% NP-40, 0.1 M Pefabloc, 1 mM DTT, complete Mini, PhosSTOP). Protein concentrations were determined using a Bradford protein assay (Biorad Protein Assay Dye Reagent, 500-0006; BioRad Laboratories GmbH, Munich, Germany). 25 $\mu$g protein were loaded onto a 12.5% sodium dodecyl sulfate polyacrylamide gel (SDS-PAGE), subjected to electrophoresis in mini-vertical electrophoresis units (Hoefer Inc, Richmond, USA) and blotted onto PVDF membranes (Immobilin-P Transfer Membrane; Millipore, Massachusetts, USA) using a Semi Dry Blotting Unit (SCIE-PLAS; Cambridge, England). The membranes were blocked with TBS tween (TBST) containing 5% non-fat milk (AppliChem; Darmstadt, Germany) for 1 h at room temperature. The primary antibodies (UROC1 1:1000; Origenen; Rockville; USA and GAPDH 1:1000; Cell Signaling; Boston; USA) were diluted in TBST, 5% BSA and applied overnight at 4 °C. The membranes were washed with TBST, followed by incubation with a horseradish peroxidase conjugated secondary antibody (anti-rabbit 1:5000; GE Healthcare Life Sciences, Buckinghamshire, England). Fusion FX Vilber Lourmat (peqlab Life Science, Darmstadt, Germany) was used as the detection system.

**In vitro T-cell proliferation and cytokine assays**

Skin draining lymph node or spleen cells collected 3 days after UCA application were cultured at 37 °C under 5% CO2 in an RPMI 1640 medium supplemented with 2 mM L-glutamine, 10 mM 4-(2-hydroxyethyl)-1-piperazineethane-

sulfonic acid (HEPES), 50 $\mu$M $\beta$-mercaptoethanol (all from Life Technologies, Carlsbad, California, USA), and 10% fetal calf serum (FCS; Lonza, Basel, Switzerland). T-cell responses were assessed by stimulating 2x105 LN cells or splenocytes on U-bottom plates coated with 5 $\mu$g/mL anti-CD3 mAb (145-2C11; Thermo Fisher Scientific, Lyon France), in the presence of 2 $\mu$g anti-CD28 mAbs (37.71, Becton Dickinson, Le Pont-de-Claix, France) for 72 hours. Proliferation in quadruplicate cultures was measured by adding 0.5 $\mu$Ci 3H-thymidine during the last 16 hours of culture, and IFN-$\gamma$ and IL-10 secretions were quantified in culture supernatants by an ELISA (R&D Systems, Lille, France).

**Statistical analysis**

Statistical analyses were performed in GraphPad Prism (version 9.3, GraphPad Software) or by using R version 4.1.0 program for statistical computing (https://www.R-project.org). The statistical tests used are specified in the figure legends.

For the microbiome analysis, ADONIS was performed by using R to determine the significance between groups in NMDS. Significance was set at $P < 0.05$. We performed the statistical analysis based on GMPR (40) normalized feature counts with DESeq2 method (41) and using the threshold of 0.05 for the adjusted P-value to determine the ASVs that were significant between baseline and 8 h after cis-UCA application (Tab.1) and UVB exposure as compared with the baseline.

**Results**

**Perturbation of bacterial communities after cis-UCA application or UVB exposure** To investigate the effects of cis-UCA and UVB on the skin microbiome,500 $\mu$g of cis-UCA (in a 70% EtOH vehicle) was applied topically to the shaved dorsal skin of C57BL/6 mice or the animals were exposed to UV radiation (Fig 1A) at twice the minimum inflammatory dose (618mJ/cm2) (Data not shown). At this dose, about 2/3 of the trans-UCA found in the upper layers of the epidermis was converted to the cis-isomer (Data not shown).

Bacterial 16S rRNA amplicon sequencing of V1-V2 regions was performed on skin swabs after propidium monoazide (PMA) treatment to remove dead or damaged bacteria. The samples were collected at baseline, 8 h and 24 h after treatment (Fig.1A), respectively, and sequences were clustered in amplicon sequencing variants (ASVs) to determine and compare the diversity and the identity (using the EzBioCloud database(17)) of the viable taxa present in the different samples.

A significant decrease in the Shannon diversity index, which measures community richness and evenness (alpha diversity), was observed in samples collected 8 h after the application of cis-UCA and the UVB irradiation as compared with the baseline (Fig.1B). An increasing trend was also observed for the number of distinct ASVs (Data not shown). A principal coordinate analysis (PCA, non-metric multidimensional scaling (NMDS) of Bray-Curtis distances (beta diversity)) was performed to confirm the differences in bacterial composition. The PCA results showed that the cis-UCA ($r = 0.39$, $p = 0.014$) and UVB-treated ($r = 0.23$, $p = 0.036$) samples were tightly clustered as compared to the baseline samples (Fig.1C). Remarkably, the changes observed in cis-UCA-treated animals were transient and the Shannon diversity index, beta diversity (cis-UCA: $r = 0.17$, $p = 0.136$) (Fig.1D&E) and the number of distinct ASVs (Data not shown), returned to baseline 24 h after cis-UCA application. In contrast, mice exposed to UVB continued to show significant differences in the Shannon diversity index and beta diversity ($r = 0.48$, $p = 0.006$) (Fig.1D&E)\.

Next, the composition of bacteria at the phylum level was examined. Most ASVs detected on the skin of naive C57BL/6 mice belonged to the phylum Proteobacteria, followed by the phyla of Actinobacteria, Bacteroidetes, Firmicutes, and to a lesser extent, Pastescibacteria. Eight hours after the application of cis-UCA or UVB irradiation, a sharp increase in the abundance of Firmicutes ASVs was observed, exceeding the abundances of Proteobacteria sequences, while the abundance of ASVs associated with the other phyla remained relatively constant (Fig.2A and Tab. 1). After 24 h, a different picture emerged: the abundance of Proteobacteria, Firmicutes and Bacteroidetes ASVs in the cis-UCA treated skin were relatively comparable, while the most abundant ASVs detected on the UV-irradiated skin could be assigned to the Bacteroidetes phylum (Fig.2B and Tab. 1).

A downstream analysis of bacterial species present in the respective samples revealed an increased abundance of 54 taxa in the samples collected 8 h after the application of cis-UCA or UVB irradiation as compared with the baseline (Fig.2C and Data not shown). Nine taxa were found in both treatments (Tab.S2), including several Firmicutes taxa (e.g. Lactobacillus johnsonii, Lactobacillus taiwanensis, Limosilactobacillus reuteri and Pantoea brenneri) and two Proteobacteria taxa, Pseudomonas fluorescens and Uruburuella testudines. In contrast, the abundance of 2 and 13 species, respectively, decreased in cis-UCA- and UVB-treated mice at 8 h as compared to the baseline and none were found in both treatments (Fig.2D and Tab.1). After 24 h, 18 species in cis-UCA-treated and 47 species in UVB-treated skin increased in abundance (Fig.2E and Tab.1), while 31 species showed decreased abundance in cis-UCA-treated and 47 in UVB-treated samples, of which 6 were in found in both treatments (Fig.2F and Tab.1). In the UVB-irradiated skin at 24 h after exposure, we detected numerous Bacteroidetes taxa, including Odoribacter denticanis, Porphyromonas sp, Prevotella amnii, and Parabacteroides goldsteinii.

Collectively, these results thus indicate that both cis-UCA and UVB irradiation treatment induces significant changes in the abundance of resident skin bacterial species.

**Certain cutaneous bacteria of C57BL/6 mice metabolize cis-UCA during growth.**

To explain the increased abundance of certain phyla upon cis-UCA or UVB exposure, we hypothesized that species that increased in abundance might metabolize cis-UCA via the histidine utilization system (Hut). The Hut system, which is commonly found in bacteria, consists primarily of the hutU and hutC genes. These genes respectively encode urocanase (HutU),(18) the enzyme that converts UCA into 4-imidazolone-5-propionate (IP), and a repressor for the transcription of the Hut system genes (HutC).(19)

To test this hypothesis, a basic local alignment search tool (BLAST) was used to search for the HutU and HutC proteins using Ensembl Bacteria (20), and most bacterial species with increased abundance were found to have both HutU and HutC 8h after cis-UCA exposure (Tab.1). In comparison, few of the bacteria with decreased abundance in the dataset contained the two proteins (Tab. 1).

Two bacteria from the data set were then selected, that may or may not express the Hut proteins, namely Staphylococcus epidermidis (S. epidermis; HutU+, HutC+; Tab. 1) and Corynebacterium singulare (C. singulare; HutU-, HutC-; Tab.S1), and we tested their ability to metabolize cis-UCA in vitro. Bacteria were cultured in the presence of various concentrations of *cis*-UCA in a liquid Luria Broth medium (for 24 h) during which their growth curves were measured (OD600); after 24h of incubation the cultures were plated on solid agar plates (incubated for 48 h) and the CFUs were counted. Under both conditions, the growth of S. epidermidis (Fig.3A&B and Data not shown) but not that of C. singulare (Fig.3C&D and Data not shown) was enhanced by the addition of cis-UCA, as measured by spectrophotometry or by counting the number of colony-forming units. Lower amounts of cis-UCA were found in the culture medium of S. epidermidis after 24 h than in that of C. singulare (Data not shown), suggesting that the former was using cis-UCA for growth. Of note, western blot experiments performed on bacteria cultures confirmed that the selected *S.epidermis* strain expressed much more urocanase than *C.singulare*, and this regardless of the presence or absence of trans- and cis-UCA in the medium (Figure 8). The bacteria were then cultured in the presence of the urocanase inhibitor, glycylglycine, together with cis-UCA. Incubation with this inhibitor diminished the growth of S. epidermidis, in a dose-dependent manner (Fig.3E), whereas the growth of C. singulare was not affected (Fig.3F). Moreover, after 24 h in the presence of the glycylglycine (Data not shown), no differences in cis-UCA concentrations were detected in the two bacteria cultures, confirming that S. epidermidis uses the Hut system to metabolize cis-UCA in vitro. Finally, we tested whether the increase in abundance of Hut+ positive bacteria also correlated with a decrease in the availability of cis-UCA in vivo. Mass spectrometric analyses of samples collected with the tape-stripping method at different time points (2, 4, 8, 24 or 48 h) after UVB exposure (Fig.4A) showed that the amount of cis-UCA detected on the skin surface increased significantly when the skin of mice was pre-disinfected with chlorhexidine as compared with controls (Fig.4B). A similar picture was observed after applying cis-UCA, although the differences were not apparent in this case until 2h after the application (Fig.4C).

Overall, these results show that certain resident skin bacteria, such as S. epidermidis, use urocanase to degrade and metabolize cis-UCA in order to proliferate.

Skin microbes inhibit cis-UCA-induced immunosuppression in the allergic contact dermatitis model.

Using the standard model of contact hypersensitivity (CHS) to the hapten 2,4-dinitrofluorobenzene (DNFB), we then tested the ability of cis-UCA to inhibit the development of an immune response to DNFB (measured as an increase in mean ear swelling in DNFB-sensitized animals (5, 21)) (Fig.5A). Similar to the exposure to broadband UVB (618mJ/cm2), cis-UCA was applied topically to mice 3 days before the sensitization to DNFB. This substance strongly inhibited the induction of the immune response to DNFB (Fig.5B). The inhibition was dose-dependent, ranging from 125 μg to 500 μg cis-UCA per mouse, and the inhibition observed was 43 and 113% of the baseline CHS, respectively, as compared to vehicle (70% EtOH)-treated controls. The inhibition also proved to be specific to the cis-isomer, as applications of trans-UCA did not inhibit DNFB-induced contact allergy (Fig. 5B).

The effects of cis-UCA in disinfected mice (i.e. cleared of skin bacteria by pretreatment with chlorhexidine) were then assessed (Fig.6A). The disinfection process completely eliminated the bacteria present on the treated site within hours of application (Data not shown). Mice disinfected with chlorhexidine and treated 1 h later with 250, 125, or 62.5 μg cis-UCA showed a significant decrease in ear swelling and a corresponding increase in CHS inhibition (89.5, 67, and 27% inhibition, respectively) as compared with nondisinfected controls (41, 12, and 0.2% inhibition, respectively) (Fig.6B, left). Interestingly, all disinfected mice that had been previously treated with cis-UCA showed a reduction in DNFB-induced flare-up responses when retested 30 days later, suggesting that they developed a sustained tolerance to DNFB. In comparison, non-disinfected controls treated with 125 and 62.5 μg cis-UCA showed no tolerance upon retesting (Fig.6B, right).

These results indicate that the local depletion of skin microbiota enhanced the immunomodulating and tolerant properties induced by applications of cis-UCA, highlighting the influence of the skin microbiome on the immunomodulating functions of this UV photoproduct.

The key role exerted by urocanase positive bacteria in inhibiting cis-UCA-induced-immunomodulation.

To test whether urocanase-positive bacteria inhibited cis-UCA-induced immunosuppression, gnotobiotic-like mouse models colonized on the area of interest (shaved dorsal skin) with Hut+ or Hut- bacteria were used. C57BL/6 mice were cleared of skin bacteria by chlorhexidine pretreatment and then colonized with S. epidermis or C. singulare for 7 days. They were then treated with cis-UCA and tested with DNFB as described above (Fig.7A). Mice colonized by S. epidermidis and treated with 125 μg cis-UCA showed the same decrease in CHS as controls that had not been treated with cis-UCA, where an inhibition of 26% was seen in both cases. Conversely, mice colonized with C. singulare, which lacks the genetic program to metabolize cis-UCA, showed a significantly reduced CHS response (73% inhibition) as compared to controls (no inhibition). Interestingly, cis-UCA tolerance (as assessed with the DNFB repeat assay, 30 days later) was maintained in mice disinfected and colonized with C. singulare (44% inhibition in CHS), as compared to their counterparts colonized with S. epidermidis (28.5% inhibition) (Fig.7B).

Finally, the ability of Hut+ bacteria to metabolize cis-UCA was blocked by the urocanase inhibitor, glycylglycine, at the time of bacterial colonization. Addition of the glycylglycine increased the efficacy of UCA-induced immunosuppression in mice colonized by S. epidermidis (44% versus 23% suppression in controls), whereas it had no effect in mice colonized by C. singulare (67% versus 73% inhibition in controls) (Fig.7C).

Overall, these experiments demonstrated that colonization of the murine skin with Hut+ microbes impaired the immunosuppressive properties of cis-UCA. Moreover, the addition of a topical urocanase inhibitor was sufficient to modulate the metabolic activity of these bacteria, leading to restoration of the immunosuppressive and tolerogenic properties of cis-UCA.

**Discussion:**

It has recognized for a long time that both trans- and cis-UCA carry out multiple roles in health and disease (6). They are among the skin's natural moisturizers and maintain the skin's acidic pH. Unlike trans-UCA, cis-UCA has also potent anti-inflammatory and immunosuppressive properties. In this study, we explored another aspect of the multiple functions of cis-UCA. We demonstrated that a significant portion of cis-UCA can be used as a nutrient source by certain bacterial species living on the skin following acute UVB irradiation or topical application of cis-UCA, thereby affecting some immune functions of the host. Furthermore urocanase-positive resident skin bacteria such as S. epidermidis metabolize UVB-induced cis-UCA which, in turn, reduces the immunosuppressive properties of UVB radiation.

Variations in bacteria communities after UVB exposure and cis-UCA application. An interesting observation of our study concerns the transient changes in the bacterial community induced by the application of cis-UCA, where after 8 h a decrease in bacterial diversity correlated with an increased abundance of bacteria in the phylum of Firmicutes, including S. epidermidis and Staphylococcus xylosus. A comparable change was also observed following UVB exposure, although an increase in abundance of members of the phylum Bacteroidetes was detected after 24 h that was not found after cis-UCA applications. Interestingly, a recent study revealed a similar alteration in human subjects, whereby a transient change (1 day after exposure) of the skin microbiome in people exposed to sunlight was reported. The abundance of Firmicutes bacteria most notably decreased and Actinobacteria increased, with a recovery of the pre-exposure abundances observed by 28 days post-exposure (22). In our study, it is likely that the initial changes observed following irradiation were triggered by the transient rise in epidermal cis-UCA, whereas the lagging increase in Bacteroidetes is likely to be the result of complementary mechanisms. Indeed, some bacterial species on the skin may be killed by UVB exposure, which is recognized to affect bacterial growth in vitro through the generation of DNA damage and reactive oxygen species, depending on the radiation dose and wavelength. Other bacterial species have developed mechanisms to protect themselves against the deleterious effects of irradiation, such as the shielding offered by the cell wall of Gram-positive bacteria (23, 24). Furthermore, beyond direct effects, the increase in the abundance of particular cutaneous bacterial communities may be affected by the release of antimicrobial peptides from skin cells. Several studies have shown that UV radiation induces the production of human □-defensins 2 and 3, ribonuclease 7, S100 proteins A7 or A12, and elafin by keratinocytes in vitro and in vivo, which are more prone to affect certain bacterial species than others (25-27).

The metabolism of cis-UCA by Hut+ bacteria. UCA is a major intermediate in the histidine to glutamate (GLU) metabolic pathway. In the skin, trans-UCA is derived from the catabolism of histidine-rich proteins such as filaggrin via the action of keratinocyte histidases. UCA is next degraded to IP, and further to formiminoglutamate (FIG) and GLU by the subsequent action of a priopionase and a glutamase.(19) Keratinocytes lack urocanase to catabolize UCA, which leads to its accumulation in the skin.(12) In contrast, the GLU pathway is highly conserved in bacteria, with more than a thousand of species expressing the Hut system. This system utilizes histidine and urocanic acid as sources of nitrogen and carbon (19). Interestingly, we recently observed that the histidine pathway is strongly affected after UV exposure in mice with normal skin, unlike germ-free or control mice in which skin bacteria were eliminated by chlorhexidine disinfection (28). The genetic organization of the Hut system and its regulation varies between species. Nevertheless, in general the Hut enzymes are activated when histidine or UCA are present in the microenvironment at concentrations that exceed those of internal pools or when the bacteria abundance is limited based on carbon or nitrogen sources (19). Here, most of the bacterial species that were observed to increase in abundance after UVB exposure or UCA application contained HutU and HutC proteins, suggesting that they proliferate in response to the transient increase in cis-UCA in the outer layers of the epidermis. However, the abundance of many bacterial species did not increase, and some such as Staphylococcus caprae even decreased in abundance after the cis-UCA application despite containing HutU and HutC. Hence, it is possible that most bacterial species that increased in abundance following the UVB exposure or topical application of cis-UCA metabolized the cis-isomer specifically, and not the highly available trans-isomer. Hug et al tested over 60 aerobic isolates from human or BALB/C mouse skin in vitro for their ability to metabolize L-histidine, trans-UCA and cis-UCA (18). In particular, they detected a cis-UCA isomerase in Micrococcus luteus ATCC strains, which allowed the conversion of cis-UCA to trans-UCA with subsequent access to the classical histidine degradation pathway (18). This finding indicates that defining the mechanisms by which some bacteria more specifically use the cis versus trans substrates should help us better understand changes and dynamics observed after UVB exposure. Another important aspect is to determine whether cis-UCA accumulation may also have participated in activating the virulence system in the more abudant bacteria, as has been suggested for bacteria such as Brucela or Vibrio (19).

The mechanisms by which Hut+ bacteria hamper cis-UCA-induced immunosuppression. To date, the exact mechanisms by which cis-UCA exerts its anti-inflammatory and immunosuppressive effects are not perfectly understood. Many studies have attempted to identify the receptor to which cis-UCA binds and its consequent downstream signaling. However, to date, several receptors have been found, such as the 5-hydroxytryptamine and/or the serotonin receptor, or the histamine-1 and histamine-2 receptors, suggesting that cis-UCA activity may vary depending on cell type (6, 13). Studies have shown that cis-UCA (i) inhibits prostaglandin E2 or TNF☐ secretion by keratinocytes or monocytes, (ii) induces substance P and calcitonin-gene related protein release by sensory nerves, and (iii) increases the expression of checkpoint ligands such as PD-L1 on Langerhans cells, thus inhibiting their antigen presentation properties.(6, 29) It also demonstrates a direct activity on splenocytes and boosts their IL-10 secretion. Cis-UCA then promotes a local and systemic tolerance state that fine-tune immune responses. In our study, we investigated the systemic properties of cis-UCA, as the latter was applied to different skin sites (back) than DNFB (abdomen and ears). The presence of skin bacteria affected the persistence of cis-UCA produced at the skin surface as a result of UVB exposure or after the topical application of cis-UCA. In addition, the presence of skin bacteria, notably Hut+ bacteria such as S. epidermidis, inhibited the tolerogenic properties of cis-UCA in the DNFB allergic contact dermatitis model. It remains to be determined how Hut+ bacteria alter cis-UCA functions on host cells. One hypothesis is that they decrease the systemic concentration of cis-UCA by metabolizing it on skin sites, thereby lessening its effect on suppressing immune function. This was tested in an in vitro experiment where cells purified from the draining lymph nodes of mice treated 3 days previously with topical cis-UCA were stimulated with anti-CD3 mAbs. These cells proliferated more vigorously than cells isolated from mice applied with the vehicle. Nevertheless, this effect was partly abrogated by pre-disinfecting the mouse skin with chlorhexidine (Fig.S7), providing evidence that skin bacteria modulate the systemic action of cis-UCA on skin immunity.

The contribution of Hut+ bacteria to control cis-UCA-induced immunosuppression in humans. Finally, as laboratory mice live in a unique habitat, far removed from the human environment, it will be important to validate the results obtained in mice and reported here, by performing experiments with individuals who receive UVB phototherapy treatments for conditions such as for atopic dermatitis, psoriasis, polymorphic light eruptions and even sunburn. In a pilot experiment, we observed that volunteers exposed to increasing doses of UV light displayed an increased amount of cis-UCA in the skin, when the skin was pre-disinfected (Fig.S8). Therefore, we hypothesize that eliminating skin bacteria before exposing the skin to UV light may improve the efficacy of phototherapy protocols that use artificial light sources or of natural sunlight. It will also be interesting to test whether using a topical urocanase inhibitor such as glycylglycine may also have similar effects, as we observed that this use restored the tolerogenic properties of this UV photoproduct in our contact allergy model.

In conclusion, our results provide a striking example of the ability of the skin microbiome to reshape host cell functions. They provide mechanistic evidence that certain cutaneous bacterial species, and notably certain Hut+ bacteria, metabolize the UVB mediator cis-UCA, influence its concentration on the skin, and thereby regulate its immunomodulating functions.

**Table 1 List of bacterial species increased or decreased in abundance after cis-UCA application and HutU and HutC protein blast results.** Bacterial species that are either significantly increased or decreased in abundance after cis-UCA application (8 h) as compared to the baseline are listed. The unique feature ID (featID), bacterial species identified by EZbioCloud database, location (gut or skin) and the presence or absence of HutU or HutC are listed in the columns.

| taxa | featID | Bacterial species | Loca tion | Hut u prot ein bias t | Hut C prot ein bias t |
|---|---|---|---|---|---|
| Bacteria\|Cyanobacteria\| OxyphotobacterialChloroplast | afd7016d407a529972c3 140fa5ac4204 | | | | |
| Bacteria\|Firmicutes\|Bacilli\|Lactobacillales\| Lactobacillaceae\|Lactobacillus | be34e9c19255551bbe1f e6d894518185 | Lactobacill us taiwanensis | Gut | - | + |
| Bacteria\|Firmicutes\|Clostridia\|Clostridiales\| Lachnospiraceae\|Lachnospiraceae NK4A136 group\|Lachnospiraceae | 852ed78727433fcc3add 09cab9ecbc9f | Lachnospir aceae Fusimonas KE159538_ s | Gut | | |
| BacterialCyano bacterialOxyphoto bacterial Chloroplast\|Triticum aestivum (bread wheat)\|‖ | 40bldl88f2eel88fedfac 4ee55d50e82 | | | | |
| Bacteria\|Bacteroidetes\|Bacteroidia\| Bacteroidales\|Prevotellaceae\| Prevotellaceae UCG-001\|uncultured bacterium | c7dd4ebeee0d53c85a8f 29aa3a2ae4c8 | Prevotella shahii | Oral | + | - |
| Bacteria\|Bacteroidetes\|Bacteroidia\| Bacteroidales\|Bacteroidaceae\|Bacteroides | b8de9599cb807310e3c 49d677d636b8b | Bacteroides caecimuris | Gut | - | + |
| Bacteria\|Proteobacteria\|Gammaproteobacteria\| Enterobacteriales\|Enterobacteriaceae\|Pantoea | 4ed831e19307b0958505 8c8c92a562e6 | Pantoea hericii JZB 2120024(T) | Gut | | |
| Bacteria\|Proteobacteria\|Gammaproteobacteria\| Pseudomonadales\|Pseudomonadaceae\|Pseudomonas | 45ea773f9a19264d2fc7 edl-l2ed23al4 | Pseudomon as syrin-gae | Plant path ogen | + | + |
| Bacteria\|Proteobacteria\|Gammaproteobacteria\| Pseudomonadales\|Pseudomonadaceae\|Pseudomonas | 5b355c35bcc760ecf6f9 b63aee56ec00 | Pseudomon as fluorescens DSM 50090(T) | skin | + | + |
| Bacteria\|Bacteroidetes\|Bacteroidia\|Bacteroi dales\|Prevotellaceae\|Prevotellaceae UCG-001\|uncultured bacterium | 49c37f30eda4459f6407 b2efdceb94f6 | Prevotella shahii | oral | + | - |
| Bacteria\|Bacteroidetes\|Bacteroidia\|Bacteroidales\| Rikenellaceae\|Alistipes\|uncultured bacterium | 0d426d96e43eea9e2299 89a8alb5ef33 | Alistipes onderdonkii | Gut | + | + |
| Bacteria\|Proteobacteria\|Gammaproteobacteria\| Pseudomonadales\|Pseudomonadaceae\|Pseudomonas\| Pseudomonas fragi | 70c7afee8864b16a6519 66ca3c13519b | Pseudomon as fragi NRRL B-727(T) | skin | + | + |

(continued)

| taxa | featID | Bacterial species | **Loca tion** | Hut u prot ein bias t | Hut C prot ein bias t |
|---|---|---|---|---|---|
| Bacteria\|Firmicutes\|Clostridia\|Clostridiales\| Lachnospiraceael Lachnospiraceae NK4A136 group\| uncultured bacter-ium | 3684ccba9d7cd2e22ee8 e11a34264861 | Clostridium fimetar-ium | **Gut** | - | + |
| Bacteria\|Cyanobacteria\| OxyphotobacterialChloroplast | eddda508a2c91423775 bc3e76605a7a9 | | | | |
| Bacteria\|Firmicutes\|Bacilli\|Bacillales\| Staphylococcaceae\| Staphylococcus\|Staphylococcus epidermidis | 18dff4bf2c40bf168e7b1 714186e6d29 | Staphyloco ccus epidermidis NCTC 11047(T) | **skin** | + | + |
| Bacteria\|Bacteroidetes\|Bacteroidia\|Bacteroidales\| Muribaculaceaeluncultured Bacteroidales bacterium\| | 7b3c456577b851f81a17 e37d0a6e2fbd | Muribaculu m spp | **feces** | + | + |
| Bacteria\|Firmicutes\|Bacilli\|Lactobacillales\| Lactobacillaceae\|Lactobacillus\| mouse gut metagenome | d80b25f0231a9d3cb538 c5d4f204214a | Ligilactoba cillus murinus NBRC 14221 | **Gut** | - | |
| Bacteria\|Firmicutes\|Bacilli\| Lactobacillales\|Lactobacillaceae\| Lactobacillus | f26e1d1072a35f4cf00c 4a22f14eb49d | Lactobacill usjohnsonii ATCC 33200(T) | **Gut** | - | + |
| Bacteria\|Firmicutes\|Bacilli\|Lactobacillales \|Lactobacillaceae\|Lactobacillus\|Lactobacillus reuteri | 2abc05a2d79e77b3685 lcdc807bl88bb | Limosilacto bacillus reuteri JCM 1112(T) | **Gut** | - | |
| Bacteria\|Firmicutes\|Bacilli\|Lactobacillales\| Lactobacillaceae\|Lactobacillus\|Lactobacillus reuteri | 5dbef09526e780d5e786 9adcf069a7db | Limosilacto bacillus reuteri JCM 1112(T) | **Gut** | - | |
| Bacteria\|Firmicutes\|Clostridia\|C lostridiales\|Lachnospiraceae | 62444cfl4386ba3a4750 84c473c13ae8 | Anaerocolu mna aminovaleri ca | - | - | + |
| Bacteria\|Bacteroidetes\|Bacteroidia\|Bacteroidales\| Muribaculaceaeluncultured bacterium\| | 6a0a53fd6aelc6f6a50ac 20c9158d673 | Paramuriba culum intestinale B1117(T) | **Gut** | - | + |
| Bacteria\|Cyanobacteria\|Oxyphotobacteria\|Chloroplast | 5986a4f202b41989673 b944cb717b779 | | | | |
| Bacteria\|Firmicutes\|Clostridia\|Clostridiales\| Lachnospiraceae | 8c85d279dc7f8e03399a 029d6837cf99 | Ruminococ cus torques ATCC 27756 | **Gut** | - | + |
| Bacteria\|Firmicutes\|Clostridia\|Clostridiales\| Lachnospiraceae | 56ea1c9010400a7c3b86 9435007flaa3 | Anaerocolu mna aminovaleri ca DSM 1283 | - | - | + |

| taxa | featID | Bacterial species | Location | Hut u prot ein bias t | Hut C prot ein bias t |
|---|---|---|---|---|---|
| Bacteria\|Proteobacteria\|Gammaproteobacteria\| Pseudomonadales\|Pseudomonadaceae\|Pseudomonas | a03d35a9ab94e254ebf4 733cc2dbl0bd | Pseudomon as azotoforma ns | **skin** | + | + |
| Bacteria\|Bacteroidetes\|Bacteroidia\|Bacteroidales \|Muribaculaceae\|uncultured bacterium\| | befeaa5d43e1e0012ace 5251d0ae925c | Duncaniella freteri | **Gut** | + | + |
| BacterialCyano bacterialOxyphoto bacterial Chloroplast\|Triticum aestivum (bread wheat)‖ | 235f3feladc2elb6f78f6 72cd6ab7a69 | | | | |
| BacterialActino bacterialActino bacterialC orynebacteriales\| Corynebacteriaceae\| Corynebacterium 1 \|Corynebacterium singulare | a33b208643a48484371 48178700164ce | Corynebact erium singulare IBS B52218(T) | **skin** | - | - |
| Bacteria\|Firmicutes\|Clostridia\| Clostridiales\|Lachnospiraceae | 99a70961b58a44e08bb 279e434a7b8ba | Cuneatibact er caecimuris | **Gut** | - | + |
| Bacteria\|Bacteroidetes\|Bacteroidia\| Bacteroidales\|Muribaculaceae\| uncultured bacterium\| | 799bbd5151676c7237fe 9648blef5dbf | Duncaniella freteri | **Gut** | + | + |
| Bacteria\|Cyanobacteria\|Oxyphotobacteria\|Chloroplast | 6d02b472cbead38c21ec 8dl028ffe050 | | | | |
| Bacteria\|Bacteroidetes\|Bacteroidia\|Bacteroidales\| Muribaculaceaeuncultured Bacteroidales bacterium\| | 2fl63ca88e79759e96f9 e31e8d56f946 | Muribaculu m gordoncarte ri TLL-A4(T) | **Gut** | + | + |
| Bacteria\|Proteobacteria\|Gammaproteobacteria\| Enterobacteriales\|Enterobacteriaceae\|Pantoea | 2el5ec0f7a8elfdfafcfd d0f62b1136e | Pantoea brenneri | **Gut** | | |
| | | LMG 5343(T) | | | |
| Bacteria\|Firmicutes\|Bacilli\|Lactobacillales\| Lactobacillaceae\|Lactobacillus\|Lactobacillus reuteri | d0abll4eebd598425fcl 9bb9096c206a | Limosilacto bacillus reuteri JCM 1112(T) | **Gut** | - | |
| Bacteria\|Proteobacteria\|Gammaproteobacteria\| Pseudomonadales\|Moraxellaceae\|Acinetobacter | 411dbcbeeb679a7ca9ba b6100bccf77a | Acinetobact er lwoffii | **skin** | - | + |
| Bacteria\|Bacteroidetes\|Bacteroidia\|Flavobacteriales\| Flavobacteriaceae\|Flavobacterium\| marine metagenome | 0b0021865el4c30b6bld 18482328d633 | Flavobacter ium succinicans LMG 10402(T | **skin** | + | + / - |
| Bacteria\|Cyanobacteria\|Oxyphotobacteria\| Chloroplast\|Triticum aestivum (bread wheat)‖ | 01046f479c85f944e8fc 1fd684218cf0 | | | | |

| taxa | featID | Bacterial species | Location | Hut u protein bias t | Hut C protein bias t |
|---|---|---|---|---|---|
| Bacteria\|Bacteroidetes\|Bacteroidia\|Bacteroidales\| Muribaculaceae\| uncultured Bacteroidales bacterium\| | b8baf9b5310df47d8811e0cfb0de9822 | Muribaculu m gordoncarte ri TLL-A4(T) | Gut | + | + |
| Bacteria\|Actinobacteria\|Actinobacteria\| Corynebacteriales\| Corynebacteriaceae\| Corynebacterium 1\| Corynebacterium pseudogenitalium ATCC 33035 | f8d75bab1cfa4b2039bd90b31f98f693 | Corynebact erium tuberculost earicum CIP 107291(T) | skin | + | + |
| Bacteria\|Cyanobacteria\| Oxyphotobacteria\|Chloroplast | e371dbd19faabcbc0635cffb4b2e745c | | | | |
| Bacteria\|Firmicutes\|Bacilli\|Bacillales\| Staphylococcaceae\| Staphylococcus\| uncultured bacterium | 8dde2713518d0be392ce930e120a60b0 | Staphyloco ccus lentus ATCC 29070(T) | skin | + | + |
| Bacteria\|Firmicutes\|Bacilli\| Lactobacillales\|Leuconostocaceae\|Leuconostoc\| uncultured bacterium | 6304c825c5ec6a938b6d0c81e30129ad | Leuconosto c mesenteroi des | Gut | - | + |
| Bacteria\|Firmicutes\|Bacilli\| Lactobacillales\|Leuconostocaceae\|Weissella\| Weissella soli | 2aca604108350511282f9a9039a8e8b4 | Weissella soli KACC 11848(T) | skin and Gut | - | + |
| Bacteria\|Firmicutes\|Clostridia\|Clostridiales\| Lachnospiraceae\|Lachnospiraceae NK4A136 group\| uncultured bacterium | 54fc3d21268f1a22e6844d98d8800d4d | Kineothrix alysoides | Gut | - | + |
| Bacteria\|Firmicutes\|Bacilli\|Bacillales\| Staphylococcaceae\| Staphylococcus | 0ff1d7346232699bbe232c0f48623d98 | Staphyloco ccus xylosus CCM 2738 | skin | + | + |
| Bacteria\|Bacteroidetes\|Bacteroidia\| Bacteroidales\|Muribaculaceae\|uncultured bacterium\| | 06e8c72e9330fdc0b08467e9f42038f2 | Parabactero ides distasonis ATCC 8503 | skin and Gut | + | + |
| Bacteria\|Proteobacteria\|Gammaproteobacteria\| Pseudomonadales\|Pseudomonadaceae\|Pseudomonas | 7e5f47e2d6b7b7f7172cf83f057984d0 | Pseudomon as graminis DSM 11363 | | + | + |
| Bacteria\|Firmicutes\|Clostridia\|Clostridiales\| Lachnospiraceael Lachnospiraceae NK4A136 group\| uncultured bacterium | 3delScffSfd6484Scc8911ba7111e3cc | Kineothrix alysoides | Gut | - | + |
| Bacteria\|Bacteroidetes\|Bacteroidia\|Bacteroidales\| RikenellaceaelAlistipeslu ncultured bacterium | 2a144159c003f4b1e56ec829el7d9318 | Alistipes finegoldii DSM 17242 | skin and Gut | + | + |
| Bacteria\|Cyanobacteria\| Oxyphotobacteria\|Chloroplast | 1dc747667842c918dacf50ff9312193e | | | | |

(continued)

| taxa | featID | Bacterial species | Location | Hut u prot ein bias t | Hut C prot ein bias t |
|---|---|---|---|---|---|
| Bacteria\|Firmicutes\|Clostridia\|Clostridiales\| Lachnospiraceael Lachnospiraceae NK4A136 group \|uncultured bacterium | b57767c6799b2968e12 54cd58deac207 | Kineothrix alysoides | **Gut** | - | + |
| Bacteria\|Proteobacteria\|Gammaproteobacteria\| Betaproteobacteriales\|Neisseriaceae\| Uruburuella\|metagenome | 568bfbc530c29a204271 8dd286c8ea7b | Uruburuella testudinis | **skin** | - | |
| Bacteria\|Actinobacteria\|Actinobacteria\| Corynebacteriales\| Corynebacteriaceae\| Corynebacterium 1 | eafl892c0f49c0afe287b f9727bb6ldc | Corynebact erium gottingense DSM 103494(T) | **skin** | - | + |
| Bacteria\|Firmicutes\|Bacilli\|Lactobacillales\| Enterococcaceae\|Enterococcus\|Enterococcus faecalis | f1a8bf0eff6b0ebd8b65c fedf584799b | Enterococc us faecalis | **Gut** | - | + |
| Bacteria\|Firmicutes\|Bacilli\|Bacillales\| Staphylococcaceae\| Staphylococcus | dc64566f68c9499bdcf2 b0632b12bc07 | Staphyloco ccus caprae | **skin** | + | + |

**Table 2: Useful nucleotide and amino acid sequences for practicing the invention**

| SEQ ID NO | Nucleotide or amino acid sequence |
|---|---|
| 1 (urocanse AA sequence) | MTDVKKSIRANRGTELECLGWEQEAVLRMLRNNLDPEVAEKPEDLIVYGGI GKAARDWDAFHAIEHSLKTLKNDETLLVQSGKPVGMFRTHPQAPRVLLANS VLVPKWADWEHFHELEKKGLMMYGQMTAGSWIYIGSQGILQGTYETFAEL ARQHFGGSLKGTLTLTAGLGGMGGAQPLSVTMNEGVVIAVEVDEKRIDKRI ETKYCDRKTASIEEALAWAEEAKLAGKPLSIALLGNAAEVHHTLLNRGVKID IVTDQTSAHDPLIGYVPEGYSLDEADRLRQDTPELYVRLAKQSMKKHVEAM LAFQQKGSIVFDYGNNIRQVAKDEGLENAFDFPGFVPAYIRPLFCEGKGPFR WAALSGDPADIYRTDALLKELFPTNKALHRWIDMAQEKVTFQGLPSRICWL GYGERKKMGLAINELVRTGELKAPVVIGRDHLDCGSVASPNRETEAMKDGS DAVGDWAVLNALVNTAAGASWVSFHHGGGVGMGYSLHAGMVAVADGSE LADERLARVLTSDPGMGIIRHADAGYERAVEVAKEQDIIVPMQK |
| 2 (urocanase nucleic acid sequence) | aagcttccgc aagccgcata tcaaattgat gtgcctgcca aagaagcggg tgtcgtctct gaaatcgtcg cggacgaaat cggccgtcgcg gcgatgctgt taggtgccgg ccgcgccaca aaagaagacg aaatcgattt agccgtcgga tcatgctccg caaaaaggtc ggcgacaaag tagaaaaagg ggaaccgctc gtaacgcttt acgctaaccg agaaaacgtc gatgaagtca tcgcaaaagt ctatgacaac atccgcatcg ccgcggaaga agcgccgaag ctgattcata cgttaattac agaataaaaa ataaagcaca tcccatgctg agcggggtgt gcttttttaa ttataggatg tgatcggctg gatattcctc ttggctgatt tttttcgctt ttgtaacatt tcttttgaaa tggtagatga gatagcaaag gatgatacaa ggaactccgc agaaaagtgc gattctttga ttgggatcaa aagcaagacc gatacaagaa gcagaacata ataagagagc ggctataggc ataagaggat aaagcggcgt gcgaaatgtc agatctttta catccccccc ttttttcaaa aatctctttc gaaacaagag ttgggacaag gcaatgctca tccagaccac gactcctgca aatccagcaa ttgcgaccat caccacatag acagttccag gggccacgat actggaaacg agagataaac aagatacagc catactgatc attaaggcat ttaaaggaat gcctttagat gtcactttct taaaacggga gctgatcatg ttttcatttg ctaaagacca catcatacgg tcgaagcat ataagcctga gttggcgaca gacagcaagg ctgttaatat tacgaaattc ataatatcag cagcgtaagg aattccgatc tccgcaaata cggcgacaaa cgggctttca atcacgcctg cgtccttcca tgaaatcaac ccggataata tgaagacagc gccaatgaaa aaaatcacgg ttctccatgc gacattacgg atggatcgcg gtatatcctt ttgaggattt gctgattctc ctgctgtaac gccgattaac tctgtaccgg aaaaggcgaa gctcacagat atcatcgcga taaaaacggc gagaaagcca tttggaaaca acccgccatg atctgtgaag tttgacaaca taggagcatc cgccgttccg tttaaagaga tcaggccaaa catggcggct cctcctaaaa tgataaataa aataatggtg acaatcttta cgctggaaaa ccaaaattca gtttccgcaa ataacttaac tgaaaatgcg ttgcatataa aaagaagggc tgcaaaaatc gcactccaca tccatacact gctgtcaggg aaccagcgct gcatgagaat accggatgcg gtaaactcag agcctaccgt caccacccaa ttgatccagt acatgatgcc gaccataaaa ccggtagatg |

(continued)

| SEQ ID NO | Nucleotide or amino acid sequence |
|---|---|
| | gaccgataaa tgttgtagca tattttttgaa aagagccggt caccgcattg gcaaccgaca attcgccgag acattgcatg acgaggtaca tcatcaaccc gccgattaca taagcgagta tcgtaccgcc gggtccggct tgatgtagcg tatagcccgt actgaggaaa agtcctgtgc ctatgacgcc tcccaatgag atcataaata ggtgcctgct tttcattgtg cgttttagct gttggctgct gttctcctga agattcactg tctcgccctc cttttagctg ccattcctta ggcagatgag tttcttatac ccaaaataca acctgccaga agatcacttg aatggggaga gtttcattcc ttttaatgcg tgtaacagaa catgagctgc agctctgctt gtcatatctc gaaaatcgag ggtagggtcg acttcaacaa tttcaatccc ggcaacattg ggctgttgtg cgatgtactt gactgcttca agcaattcgt ctgtatagag gccgcccggg ccaatcgctg gacagccggg tgcatgggac tggtctaaga catccatgtc aacagatata aaaatgaaat ctgttttatc ttgaacaaca ggcaaaatct cttttatcgt tgggattagt ccttttttccc ggatcatgtc catcgtgtga atgttcacgt tatgttttt tgcatatgct tcataggcct ggctgttgct gaattctctg atacccagct ggatcaaatg ttgaccttca atgatctctt catccaaaag gcggcggaag ggggtgccgt ttgtcggccc cccgtcctcc gtattgcgaa catcgtgatg ggcatcaaat tgaatcaccg ctgtcgttcc ttttgtttgt gctatcgctt ttattgtgga gtagctgata gagttatctc ccccaagtat gagcggcacc caatccggat gatccgaaag cagagcgtgc atggtttgga aaatgtgatg atgtgacttt acgatatcag tgacatgaat atcgatatcc cccaggtcat atagaagctc tgatacgacg tgctctccaa gctcagcaga ataggctgat gaatgtttca gagcctggcg tattgttccg ggtgcaaagg aagcgcctga atgactgatg gaggattttg ataggggggac acctatcaac gccggtccct taatgtcctg gccgtcccat gttgcgatca ggtcgctcat ttttgtgaca tcccggtctt taaacgaaga tccggcctct ctgagaaaag ggtacttatc caagaatggc tccctccctg ttgacaacaa ttgttccatt tttcatgact tgatgtacat gattgacccc gtagtgatac ggaatataca tatagttcgg ggcttgccaa atgacaagat cagctgagcg gccagctttg agctgaccgg cttcttctcc ttttcctatg gcatatgcgg cattgactgt gaccgcgtgc catatttctt ctgcagtcat tttcagatga agcgcggcaa tggacataat cagctggata ttttcagtcg gcgagctgcc aggattaaaa tccgttgcca agctgacaca tacaccttca tcaatcatag ctcttgccct ggcgtatgtg cttttgccaa gataaaatgt cgtgcccggc aagaggacgg cgatcgttcc tgcctcagcc aatttcttta tgccttcgtc cgatgtacct accaaatggt cggctgagac ggcctttagc ttacctgcta attcagcacc tccaagcgga tcgatttcat ctgcgtgtat ttttaatccg aagcctgcct cagctgcttt ttgtaaataa cggcgggatt ggctgactgt aaaaacgcct gtttctgtaa aaatatcagc aaagctggcg agctcctgct ctttgatttc cggcaaaagg gaaagcattt gatccagaaa atcatctgga tcgttttgat attcgggagg aatcgcatgg gctcccataa atgtggaaac aaggtcaaca ggctggcttt catgcagttt ctttgcgaca cgcaactgtt tgagctctgt ctctttttcc aagccgtagc cgcttttcac ttctgctgtg gttgtgccgt aggacagcat tctttgcaga tgaaaatggg cttttttgcag cagctcttct tctgaagccg ccctggtatc cttgactgta gacagaatcc cgccgccctg cgccagaata tccaaatatg aaatgccctg aagcttaagg ttcatctcct tttcacgaga tccgccaaat acaagatggg tgtgcggatc aacgaggcca ggcgttacaa ggcgtcccga acagtcaatg atttcgtctg cctcgtaacc ggcttcagcc ccttttttgtc cggcgaacac aattttctgt tcatggatgc cgacaacagc gtcttcgatc acatgaagat cctgcatgct tttgcctgcc cgcggaccgc tgctttccat cgtcaaaagc tgaccgatgt tgatgagaat cgtatcaatt tgctttggca tgtcatcact ccttttatttt tgcatcggca cgataatgtc ctgttccttc gctacttcaa cggcacgttc ataacccgca tccgcatgac |

(continued)

| SEQ ID NO | Nucleotide or amino acid sequence |
|---|---|
| | ggataatgcc cattcctggg tcgctcgtta    agacccgcgc gagccgctcg tcagccagct cacttccatc ggccactgca accatgccgg   catgaaggga atagcccatt ccgacgccgc cgccatggtg gaaggaaacc cagcttgcac     ccgccgccgt gttgacaagc gcatttagca cagcccaatc accgacagcg tcacttccat ccttcatcgc ttctgtttcc cggtttggag aggcgacaga cccgcagtcc aaatgatctc tcccaataac gacaggcgct tttagctccc cggttctaac cagctcgtta atggcgaggc ccatctttt tcgttctcca tatccaagcc agcagatacg ggacggcaag ccttggaagg tgactttttc ctgtgccata tcaatccagc ggtgaagggc tttatttgtc gggaacagct cttttaataa cgcatccgta cggtaaatat ctgccggatc tccggaaagc gccgcccagc ggaacggccc ttttccttca cagaataagg ggcggatgta ggcagggaca aaccccggaa aatcgaaggc attctccagc ccttcatcct ttgcgacttg ccggatattg ttgccgtagt caaatacaat agagcccttt tgctgaaagg ccagcatggc ttcgacatgt tttttcatgc tttgcttcgc aaggcgcacg taaagctctg gagtgtcttg acgcaaacgg tcagcttcat caagggaata gccctcggga acataaccga ttaaagggtc atgggcagat gtctgatctg tcacaatatc aattttgacg cctcggttca aaagtgtgtg atgcacctca gctgcgtttc cgagaagtgc aatggacaaa ggttttcctg ccagctttgc ctcttcagcc caagctaagg cttcctcaat cgatgctgtt tttcgatcgc agtattttgt ttcgatccgc ttgtcgatgc gtttctcgtc aacctcgacg gcaatcacga cgccttcatt catcgtgact gacagcggct   gggcgcctcc cattccgccg agacccgctg tcagcgtcaa cgttcctttt aagctgcccc cgaagtgctg tcgcgccagc tcagcgaagg tttcataggt tccttgcaat atgccttgtg agccaatgta aatccagctg ccagccgtca tctgtccgta catcattaac cctttttct caagttcgtg gaaatgctcc cagtctgccc acttcggaac aagaacagaa tttgctagga gcacgcgagg ggcctgcggg tgtgtacgga acatgccaac gggctttccg gattgcacga gcaatgtttc atcatttttt aatgttttca aggaatgctc aatggcgtga aaagcgtccc aatcccgtgc ggctttgcca atcccgccat atacgatgag gtcctccggc ttctcggcga cttctggatc aaggttgttt cgaagcatcc gaagcactgc ctcttgctcc catcctaagc attcaagctc tgttcctctg ttggcacgga ttgatttttt tacatcagtc atatgttcat ccccccttagt tctttattct gatgatcagg tattaaagat tctttcttca gccagtcagt caagcgttca atgtcatagg aaaaaacacg gtcctgctga atggacggca cgactttct catttcttga aaaagctgtt tcgtatagga cgctgcatgt tctatgcctc tgtattcaac agcctgcaat gcgcaaatgg cttcgatcgc aatgactctc cttgtatttg cgatcacctg ataagcatgc cgagcggcga tggttcccat gctgacatgg tcctcttggt tggcggatga cggaatggaa tccacacttg caggatgggc cagtgtttta ttttcagaaa cgagtgaggc cgcggcgtac tgcatgatca ttgcaccgga ttgcagaccg ggatgagggc tcaggaacgg cggcaggtca ttcagctgcg gattcacaag tcgttcgatc ctacgctctg cgatattcgc aagctctgat attgcgattt tcagaaaatc catggcaaag gcgattggct gtccgtgaaa gtttccgccg gaaatcactt tgtcaccgtc attgaaaatg agtggattgt ctgtagctgc gttcatttca atctctaatt tttctttgac atatcctagt gtctgccaag tcgcaccgtg tacttgcgga atacagcgca atgaataggc atcctgtacc cgcagttctc cctgggaggt tgtcaggccg ctgtcagata gataaaagcg aattcgttct gcgacatcga tctgctcctg atagccgcgg gctaagtgaa tatcctcgtc aaatgcatcg ataattccct gcaacccctc aatcgttaag ctggcaattc gttctgtttg ataggctagt ttctcagctt cgatgtaggc gaccacccc attgcagtca tggcctgagt cccgttaatc aaggcaagcc cttccttgga tgtcagcgtt accggttgaa tacctgcttt tttcaagcct gtcattgccg gcattctttc cccctcaaaa |

| SEQ ID NO | Nucleotide or amino acid sequence |
|---|---|
| | aagacttctc cttgtccgat cagcgcaagc gccaagtggg aaagcggcgc cagatctccg cttgcgccaa gtgatccttg ctgagggatg acaggatgga cgcgtttatt taaaaaagca agcaactgct cgatcagctc agcgcgtaca ccggaaaagc cttttaacag cgcattggcg cgcaaaagca gcattgccct ggaaacacac tcaggaaaag ggtcgccgac tccgcaggca tgagaaagga ttaaattcag ctgcagcgcc gccgagtctt ctttctgaat caggacatcg ctgaatttgc caaatccggt gttgatgccg tagattgtct tttcgtcgcg tacaatccgt tccactgctg ctctgctttt tttcacgcgc tccatacttt cctcagaagc cgctgcctcc tcaaaatcaa acagcacacg agcgacgtcg gctgttgtca atgaagaacc gtctaaagtc accataagcc caactccttt ttctttactt tggtaaagcg cccatacaaa aaggagctat gctgtgaact tctgttttca cgcatagccc cctatcgtct aagatctatg ggctgtttca tatgtgatta attccaacgc cgaatgtttc atgctcaaga cctttaatcg gcgccccgat tgttccgtaa agcgagacag cgatccaatc gccttccgct tcactttcat aaggatttcc cctcaaaacg gcaaacctca atcccaccgt ccgaagcagc gatcccagca gcatttcacc tctggtcacg ccatgcaaag cctccatcgt cgcatgataa agcgcatgtg actcccgata accctcagat tgaatgacac cgctctttt ggaagcggtt tcaattgcgg ctactacttt atgtgcgtcc attgatccta ccttgccaag acagaccttc catccgtctc gctccagctc ctcaacctgc gtactttctt ccgcctcatt cagcagcagg agaacagaca gccggccgat ccgacgctct ttatgcagtg tcatatgaat cactcaattc ctaaaaattc tgataactat taactgagta ttatattaat tggttcagca gcagaagtca aaaggttttt ttatagtctt taacaagtta gattgctatt gtaatagcgt gacgcagaaa gcagaacagg ttttttcagc gattgcacac gatatccaga caaggaggca agcgccgctg tctaactctc aatatgcgtt tactaagtcg tattttttcgc aaggcgtttt ataaagagcc agccgaatga ctctggtgtt gacgggcaat caggcatcat cttaaaaaac gccgacaacg aaatggctgt atcacagttg atgcaaaacc tgacaaagca gttattacat gtacgcgaaa gaaaaacaga cgtgatgcaa gaaggagaaa cagcaaaaga gttgaactat gaaggagagg atatgcaagc aacaagttct gcgcaaaaca gacagacatc agtctgacta tgtaagtgaa gtgagaaatc acctatctgg atgaatatga aaacattcgg ctgtttttaat aggccggggt atcttcatag agaaatgtaa gcgtttaaaa tttaataaaa aagagaggcg aattgtttag atgttcaacc gattgttccg tgtatgtttt cttgcagctc ttatcatggc cttcaccctc ccaaactccg tctatgcaca aaaacctata tttaaagaag tcagcgtcca tgacccttcc atcattgaaa caaacggcac tttttatgtt tttggatctc atctcgcttc agctaaatcg aatgacctca tgcaatggca gcagctgacc accagcgtaa gcaatgacaa cccacttatc ccgaatgtct atgaagaatt aaaagaaacc ttcgaatggg cgcaatccga tactctttgg gcggctgacg tcacgcagct ggcggacggc aaatactaca tgtactacaa cgcctgccgc ggcgattcgc caagatccgc catgggtgtt gccgttgctg acaacatcga aggcccatac aaaaacaaag gcatcttttt gaaatcagga atggaaggca caagcagcga tggaacaccg tatgatgcaa ccaaacatcc gaatgtcgtc gacccgcata cctctttttga taaagacggc aagctgtgga tggtgtacgg atcatactca ggcggcattt tcatacttga aatgaatccg aaaacgggct ccccgcttcc tggacaggga tatgggaaaa aactgctcgg cggcaaccac agcagaatcg aaggcccgta tgttctctac aatccagaca cccaatacta ctacttatac ctatcatatg gcggtctcga tgcaacaggc ggctacaaca tccgtgtggc ccgctccaag aaaccggacg gcccttacta tgatgcagaa ggcaatccaa tgctcgacgt ccgcggcaag ggaggcacat tcttcgacga ccgttccatc gaaccatacg gcgtcaaact catgggcagc tacactttg aaacagaaaa |

(continued)

| SEQ ID NO | Nucleotide or amino acid sequence |
|---|---|
| | tgaaaaaggc accgggtacg tctcaccagg ccacaactcc gcatactatg atgaaaaaac cgggcgttcc tatttaatct tccacacccg cttcccgggc aggggtgaag aacacgaagt cagagtccac caattgttca tgaacaaaga cggctggccg gtagcagcac catatcgtta tgcgggcgaa acattgaaag aagtaaaaca aaaagacatc acaggaacat acaaacttat ccagcacgga aaagacatct cagccgacat caaacaaacc atcaacatcc agctcaacaa aaaccacacc atttccgggg aaatgaccgg aacatggagg aaaaccggaa aaaacaccgc cgacatcacg ttagcaggca agaagtataa cggggtattt ttacgccagt gggattcggt gagagagaag aacgtcatga cgtttagtgt gctgaatacc agcggtgagg ctgtatgggg atctaaataa gatggagaag cgccttcgag ggaaggtgtt tttttatgag ctttattgac tataagaaac agttaagtgg gaggaaaaat tagaactgga ggagagatta aaggcttaaa taaaaagtcc ctactatgat tgttttctcc tttcataggg tgatgagatc gtattttttag caatttagaa ccagttctat acctctcaag acaatccttt ttacctatttt atccaatatg acccatcatg taatatgaaa atagtctgta ttgtaaagaa tttacacagt ttgatagaaa cacgaggtgt tgcggcatgc atagtgaaat gttgttacat tcggtaaaag ccgacttaca tgaaaagcag gaacagatcc atcaattaaa acgagtgctg cacgaaatta ggcaaatcaa acacgatttc tctgaggccc agcatctcat tcaccggcct catcttaacc gggaggcatg gagaggaaca catgctgagc ggtttgaaga tattcgtgaa ggaatgaaca aagcatatca gcaaatcaaa agcgatcaag tcagcggaat catcgaaagc atagaaggga aaatccactc attagagggc gatgtgtatt ctataagacg ccaaatcacg aggatagagc atgaaataga gaaggaaaaa cataaaaagt gaggtgagca caaatggcac aggaaattaa aatggtgtat gacacagtga agcaagggct ttctcagctc aagaattcag ctgaactcaa gtcctcattg cccggtcatc tctcaggcag aaaccatttg aatgttgtga agtccatcga gcaattaaat aaggacatca aagagctcac tgaagcatat gcgtctgttc tggccaagca tatcgcacaa acggaaagcg cggtaaacgc catgaaggaa acggacgaaa acatatcatc ttccatgaaa taaagcgcgg gaggacaagg gattgaaaac attagatgtc cacgctcttc acgagggcat tcagcatacg attgagaaac tggacaagca aaaacaacag ctggagaagc tggaaaaaag cgttgagcat ctggcaggca tgaaggatgc gctgaaagga aaaggcggag atgccatccg gacctttac gaggagtgcc acaagccttt tctcctttttc tttggcatat ttatcgacga atacaaaaaa gtgctgaaac aaacacagca tgcgatttcc tctgtagaat ctaactccca cggcatgatc gcagaggctt ttctctcaca cgatgcgaga catggggtca agcatgcacg agaagtgaca gagcagctca ctgacgcagt caatcgccag acatcagcca tagatcacat cgtcagcctg ccgacagtca atgattcctt ttttcggatg gaaacagaac aggctgagcg gctgatttca gacaccctga ataagctctt tcaatttgac ggccagcaaa cccaagcact agaagccgga aaatcagatt ttcaaacgat gaaaaaatac attgatcagc tcgaaaccat gtacaccggc ccgaaaattg aaatcacagg ctacaaaagc ggctccatcc tgaaatcaca ggaagaagaa aacatcaacc aaatcttcgg cgcgattaac ccgcaaatga agcaggcgga tgattcaccg atggagatga tgctgaagaa attagccgag aatgaaaagt ctaaagtgga ttcagttgtc aaaacgggtg attctaaaaa ggtaagtaaa aatatcatcg ttatcaatgg taaagtgtat aatacgagtg aacatagaga acatataaaa cctgatttct caaatgcaga ggttaagcaa gtcgtttata atgatacact ttacaatgta tacatatctg gtaatgacat gaagcttgaa cctgtcgtct cgctttcgga cattaaagtg gatgagaatg gatatgtgaa aatttttagag acggctgtag aacttaccgg cgtgtatgat ctatttaaag cagccacggg cagagatccg |

(continued)

| SEQ ID NO | Nucleotide or amino acid sequence |
|---|---|
| | gtatcaggcg aaaaagtcac agggaaagac cgtgttgttg cctccataaa ctcagtacca tttgccaaaa tagctaagtt agaaaagtta atagatatta ataaacttat taataatggg gaaaaggcta agaaggcctc ggaagttaaa aacgttgcta aggataaagg gaaaattgcg aatgatgtaa gtggatctgc taataaaatt aattctgatt taataaaaaa gtatgctaga gatatagaac aacgcacagg tagagaatta cctaaaaatc aaatagataa attaaaggaa gcattaagaa ataaagaata taaaaaaatg tcaccaatag agactgcaaa acacaggact aaatttgata aagtaaaaaa taaagttata aaagaatggg aagaaacac tggacaaaaa tggcctgttt ataaggaaaa tgttgtatct gaaaaaacag gtaaaattat tagaaaaaaa ggggataagt atgatgctca ccatataatt gaaaatactt ttggcggaaa acatgaatgg tggaatatgc atccagcaaa atttcctaac gagcatcaag ctgggattca tggtacaggc tcaccagcaa atgaattatt taaaggaggc aaaaaaaaat gagttataat tttataaaat caaacaaaga aaatgatttt tatccagtga atcaaagtga gattgaagag gttgaaaaaa atttaaactt aaaattacct agtgaattag tgaattttta tttagaagtt ggctatgggt ttattaaagg atcagagttt aatactaatc gcattttgga tccatattct gtaagagact ttcgattgag gataaatgac tttgaatttt atcctgacat agaaatatat gatgagtttg agaatgataa attaatattt tttgaaggta gtgaatcggc attaatgtca attgaattaa atgataataa taaaaatccg atttactatt atgatattca aattgcaact tctcttaccg agttcttaag aaaaatagaa gagaatgatc agtattatct tgatttatta gatgatgaat gattttaaca ttatcccgtt acctctccct taaaggagca agatgacatg ttattgatca ataaacagaa ggaccctgtt tttcagtttt tagctggaac attccatcaa gatattgaaa ctcctgatga cgctatacaa gagttattaa tagaggaaag taaggaatat ttggaggatg ctattgtctt tttaacggat tttattgaaa gtgagcattc agacaataag aaaaacgatt acattcaatc ttgcgcagat ggcgtgtatc ttcctgcgtt taatttagaa ccaatagatt ggctaaagaa tgtaatagta cagataaaga agtctttaaa aaaatttaaa cggtaacgat tcataacctt gattggaaaa aatgcctttc aaggttttta attacaataa ttttttattg aagttaatac taagttcaaa tctttatgat tacttttgtt ctagaaacga tagtcaacac caaaaatgaa agcgttgaca tctcacgaat ctagtgctaa agtataccta caaacaaata aaacgacccg tcaggattgt tactgcgaaa gcaggcaaaa cctaaatggt gcaggtgcag gaggagactc tcgcaatctg tgtcatttag gtttttttat tctcttttag gaggtgatgc agcatttgat acaaaaaaag ctggtgaaag gcggccacct ttcatccagc agtcccccca taaaaaggag gtatacacat ggattatgat aaaattatcga aggacatatt acaactcgta ggcggtgaag aaaacgttca gcgcgtgatc cactgcatga cgaggctgcg ttttaatctt catgacaacg cgaaggcgga tcgcagtcag ctggagcagc tgcctggagt gatgggcacc aatatcagcg gcgagcagtt tcaaatcatt atcggaaacg atgtgccgaa ggtgtatcag gcgattgtgc ggcacagcaa tctcagtgat gaaaagagcg ctggttcatc ctctcagaaa aagaatgtgc tgagtgcggt ctttgatgtg atttctgggg tgtttacccc tattctgcct gcgattgcgg agcgggggat gatcaaagga cttgtggcgc tggcagtcac gtttggctgg atggcggaga agagccaggt tcatgtcatt ttgacggctg tcggtgacgg tgcgttctac ttcctgccgc tcctgctagc gatgagtgcg gcgagaaaat cggaagcaa tccgtacgta gcggctgcca ttgcggcggc gatcctgcat ccggatttga cagccctgct cggtgcgggg aaaccgattc cctttatcgg gcttcctgta accgctgcca cttattcgtc aacggtcatc ccgattttgc tgtcgatttg gattgcttca tacgtggaaa aatggattga ccgttttaca cacgcatctc tcaaattgat tgtggttccg acattcacgc tattgattgt cgtgccgctg acattgatta cagtcggtcc gcttggcgct atttaggag aatacttgtc ttctggggtc |

(continued)

| SEQ ID NO | Nucleotide or amino acid sequence |
|---|---|
| | aattatttgt ttgatcatgc agggcttgtg gcaatgattt tcctggcggg tacattctct ttaatcatta tgacgggcat gcactatgcc tttgtgccga tcatgatcaa caacatcgct caaaacggcc acgattattt actgccggct atgttttttgg cgaatatggg gcaggcggga gcatcatttg ccgtcttcct tcgctctaga aacaaaaaat ttaaatcact cgcgcttacc acaagcatca cggcgctgat ggggatcaca gaacctgcga tgtacggcgt gaatatgcga ttgaaaaagc cgtttgcggc cgctttgatt ggcggtgcag ccggtggcgc gtttttacggc atgacgggtg ttgcttccta tatcgtcggg gggaatgcag gtctgccgag catccctgtc tttatcggcc caacgtttat ttacgcgatg atcggccttg tgattgcgtt tgcggcggga acatcagctg cgtatttgct tggattcgag gatgtgcctt ctcaccgcag ccagcagccg gcagtgcatg aaggcagcag agagatcatt cacagcccga ttaagggaga agtgaaagcc ttaagtgaag tcaaagacgg agtgtttttct gctggggtca tgggaaaagg atttgccatt gagcctgaag aaggagaagt tgtttcgcct gttcgtggaa gcgttaccac cattttttaaa acaaaacacg caattggcat tacgagtgat cagggagctg aaatactcat acacattgga ttggacacgg taaagctgga gggacaatgg ttcacggctc acatgaaaga aagcgacaag gtcgcaccgg gagatccgct cgtgtccttt gatctggagc aaatcaaagc cgcgggatat gacgtcatta ccccggtgat tgtgaccaac acagaccaat attcgttttc gccggtgaaa gagatcggta aagtgcagcc aaaagaagcg ctgcttgctt tatcttgaaa aaactaatgg ggtgatcaat atgagttcaa atgaaaaacg atttccagaa ggattttttat ggggcggcgc agttgctgcc aaccaagtgg agggtgctta taacgagggt ggtaaggggc tttcgacagc tgacgtatcg ccgaacggga tcatgtctcc atttgatgaa tcaatgactt ccttgaatct gtatcacaac gggattgact tttatcatcg ttataaagag gatatcgcct tatttgcaga aatgggggattt aaggcgttcc gcacatcgat tgcgtggacg agaattttttc cgaatggcga tgaagaggag cccaatgaag aaggcctcag attttatgat gatctgtttg atgagctgtt aaagcatcat attgagcctg tcgtaaccat ctctcattat gagatgccgc tcggtttagt gaaaaaattat ggcggctgga agaatcgcaa agtcatagag ttttatgaac gctatgccaa aacggttttc aaacgctatc aacacaaagt gaagtattgg atgacgttta atgaaattaa cgtagtgctc catgcgccat ttaccggagg cggtcttgtt tttgaagaag gagaaaacaa attaaacgcg atgtaccagg cggcacacca ccaatttgtg gccagtgctc ttgctgtcaa agcgggtcac gacatcattc ctgattcgaa aatcggctgt atgatagcgg caacgacaac ctatccgatg acttctaagc cggaggatgt cttcgcggca atggagaatg agcgcaaaac acttttcttc tcagatgttc aggcaagagg cgcatacccg ggatatatga aacgctatct ggcagaaaac aatattgaaa ttgaaatggc tgaaggcgat gaagagctct tgaaagaaca tacggtggat tatattggat ttagctacta catgtcgatg    gcggcgagca cagatccgga agagcttgca aaatcagggg gcaacctgct gggcggagtc aaaaacccttt acctcaaatc atcagaatgg ggctggcaga ttgatccgaa aggcttgcgc atcacgctca acaccttata cgaccgttac cagaagccgc tgttcatcgt tgaaaacggc cttggcgctg tagacaaggt tgaagaggac ggcacggattc aggacgatta cagaatcaac tatctgcgtg atcatttaat tgaagcgcgc gaagccattg cggacggcgt tgaattaatc ggatacacat catggggggcc aattgaccttt gtcagcgcat ctactgcgga aatgaaaaag cgctacggct tcatttatgt agaccgggac aatgaaggaa atggaacatt taaccgcatc aagaaaaaaa gcttcaactg gtatcagcag gttatcgcca caaacggaga gagtctctga caatttttttg aaaactcatg cgcttcaatt gacaataacg aaatgcaggc ggacaataaa agagaaagat gaaccaccca cagtccgaaa ggcggggaaga |

| SEQ ID NO | Nucleotide or amino acid sequence |
|---|---|
| | gcatgtttaa caaaatgtta gtagcgattg acggatcaga catgagtgca aaagcgcttg atgccgcagt gcatttggca aaagagcaac aagcggaact aagcattctt catgtgggga gagaagccgt cgtcacgact tcttctctga cgggaattgt ctatgtgcct gaacacttta ttgatgaaat caggaacgag gttaaaaaag agggcctgaa aatccttgag aatgcaaaag aaaaagcagc cgaaaaaggc gttcaagctg aaaccattta cgcgaatggc gagccggcgc atgagatact aaatcacgca aaagagaaag gcgtcagcct gattgtagtt ggcagccggg ggatcagcgg cctgaaagaa atgatgcttg gaagcgtcag ccataaagtg tctcaattat caacatgccc ggttttgatt gttcgataga tttgtaaaaa aagcgccctt gggcgctttt ttttatttag aatagaagtg ttataatatc tacatgtaaa aaagaggaat tctatgtgta gatttctgaa ttcacaaaaa acagtttatg ctatgcagta ttggaggtag aatacgtttg atgcaacagg atccttccat atcgtcaaaa caaaaatcaa cagccgtaat caaaatggtc atctctatgg tgattttcgg ctccatcggc tttttctcag agcataccaa tctgccgtca tttgaattgg tctttgtccg ctgcctttgc gcgacgctgt ttttaggctt ttgctggctg gcgtccggcc agtataaaac tgaaaaatgg agcaagagag acgtgctgca acattggcg tgcggtttct ttcttgtatt caactgggtg ttcttattta agtcttttga agaaacgtcc gttacaattg ccatctctgt ttatcatcta gcgccggtgc ttgttcttct tctggggagc ttcttctaca gagagaaatt aaatgtgatc tccgtcagct ccattatcat ctgctttctt gggactgcgc tgatttccgg gattaacggc agcacatcat tgacacagct gatgggatca gggatcatat gggcagttct cgccgcactg ttttatgcgt tcaccacttt attgggaaaa ggcattcaca atctcagtcc ttacacgacc accttcttgc agacaggttt aggggttatc atcctgattc cgtttattca ctttggcgct tttgcggacc tatctcaggg aaactggatt atggtggtat ctaccggcat catccatacg ggtattgtat atttgctgtt ttttgacagt ttacgatttt tatcaacgaa atttatttca atcattgtat ttctcgaccc cgctgtcgcg atcgtgctcg ataccgtctt cacaggcttc cgccctgacc tctatcaaac gcttggcatc gtaatgatct ttgcgggcat ggccttgacg cttgtcagga ggcaggggaa ggcgaatgtg acagctgagg gtacggatat tgaacaaata caataaaaaa tgtaaaaagg cctatgcggc ctttttttgt tttaggtcaa ttgactctcg ctaatcctta aaataagata aattttctag aaaaatattg taatgatatt tcagtctagt taagattatt gagtaaatat tacttttatt acaaaaggag agaggaaatg aaaaaaagaa agaggcgaaa ctttaaaagg ttcattgcag catttttagt gttggcttta atgatttcat tagtgccagc cgatgtacta gcaaaatcta cagaagaaga gaatggaaac cgtattgctg ccgatgatcc agaagaaact cttcagaaag aacaaactga ggaagctgta ccttttgatc caaaagatat aaacaaagag ggtgagatca cctctgaaag aacagaaaat acaaaactct attatgaagg tgacggtgtc tataaacagg aagtatacct tgatcctatt catacaaagg aaacaccgga tgccgactgg gaagacattt ctcctgagtt aaaagagtcg acaagcaaac aagtcgagac agagaacgct attttgaatt cagattttca aaagcaaatg aagaacgggc tttatgcgac ttttgagcat aatgaccata aggttacata ttcactagca gaagcgaaag gcccgaataa aacttcactt actccgaaag ataacctcagc agattataag actgacagta atgagattgt gtatccggat gtgtttccga atattgatct tcagactttc acattcaatg aaaacataaa agaagattta gtccttcatc agtataatgg atacaacaca tttacatttc agttaaaaac cgatttgcag gctaaagaac aagaggatgg atccattgat ttttctgatg aaaaaggaaa agttgttttt tctgtgccta gcctttttat gacggattca aaactagatg agctttccgg cgaggttgaa cggtcagaca aagtcagcta taagctggaa aagaatgaag aagggtattt gcttcatctt acggcagacg agaactggct gaaagaccca gaacgcgtat atccagtatc tatcgacccg tcaacttctt |

| SEQ ID NO | Nucleotide or amino acid sequence |
|---|---|
| | tatctgtttc atcggatacg tttgtcatga gtgcctaccc gacgacgaac tattcggcct cctcacaaaa atgggatgct aatttaaagg cctatgtact gaaaacgggc tattatgaca aaacaacagg cacaaactat gcgttcatga aatttaacaa cctaaagcca attcaaaata tgactgtcac taaagctaca ttaaaaacat acgtagccca ttcctattat ggaacaaagg caaccggcct ttggctggat acggtcaaca gcaattatga taatgcaaaa gtgacatgga acaccaaacc ggcatctaaa aatatcggaa aagccgacgt ccataaagga cagtgggcga gttatgatgt gaccgctgct gtaaagtcat ggaactccgg cggggccaac tatggatta agcttcatac aaacggaaac ggcaaggaat attggaaaaa actaatttct tcagcgaact ccgccaataa accatatatt gaagtgacgt ataccattcc aaagggaaac acacctacaa tcaaagccta tcacaacggc gattccactg gttatttcga tatcagctgg aaaaaggttg aaggcgcaaa aggatataag gtatggattt acaatggtaa ggaatatcaa gcgatttctg caggcaacgt cacgtcgtgg agcacaaaag gtaaaaaaat ctggcctacg agcgcagaaa ttgcctccaa gagatataaa ctgcatctgg acggcaagga tggagctgaa ttggcattag atccatcccc ggtctataag aattccggcg gaagctatgc tacaagcaaa aactattgga ttggcgtcag tgcgatattt gaccaaggtg aaggagcgat gtccgcaccg gcaaagcccg ttatccctaa tgtaggaaaa gcacaagcac cgtcagcaaa gggctacaat aatgggaatg ccacaggata cttcgacctt tcttggaaag ctgtatccgg tgcaaccggc tataaggttc aggtgttcaa tgggaaaggc tttgagacac ttgatctcgg aaatcagacg tcttggacca caaaagggaa aaagatctgg ccgacaagtg cagaaatcaa ggcgggaaaa tatgctcttc acctaaagga cggaagcgga gcagagctgc cgatcaatcc cggaccaacc tataaaaacg ccggggagga tggagccaaa agaaattact cgtttaaaat tatcgcatac aacaaagacg gcgaagccat tgcatccccg gctgctaccc cggcacttcc tgatatcgct aggccaaaga atgtaactgg ctatttgtat acaaatacaa aatcgagtca aacgggttat gtgaatttga tatgggaaaa ggttcaaaat gcgaagggct ataaagttaa catttacaac ggtaaagaat atcagtcctt tgatgttggt gatgctgatc attggacaac ccaaaataaa aacatctggc cgacttctga ggaaatcaaa gccggaagct ataagcttca tacagatgga aaaggcgggg aattagccct cgatccatca cctgtctata acaatgcaaa tgggaattac aaagggaaga agaattattc tttcacgctt gtcgcctatg atgcgaatgg tgaaacgatt ccaacggcgc cctttaaccc aacgttccat gaaggcgcag aattcctagg tacagaagag tactggtcta tcattgatat cccaagcggc cagttaaatg gtgcgacagg caatgtcatt gtgaatgaag aagatttgtc aattgatgga cgcggtccgg gacttggctt atcgagaacc tacaacagtc ttgacagctc tgatcactta tttggacaag gctggtatgc tgatgcagaa acatctgtca tctcaacgga tggcgggca atgtatatcg atgaagacgc tacgacacat cgattcacga aaaaagccga tggcacgtat cagccgccga ccggtgtata tcttgaatta acagaaacag ctgatcagtt catattaaaa acaaaggatc aaaccaacgc ttatttcaat aaaaaaggcg gcaaactcca aaaggtagtg gatggccata acaatgcaac tgtctatact tacaatgaca aaaatcaact gaccgccatt acagatgcat caggccgcaa gcttacattt acctatgatg aaaacggcca tgtgacgtcc atcacaggac ctaagaataa gaaagtcacc tactcatatg agaatgattt gctgaagaag gttacagata cagacgggac tgtcacaagc tatgactacg atagtgaagg gcgccttgtc aaacaatatt cagctaacag taccgaagca aagcctgtct ttaccgaata tcagtatagc ggtcaccgtc tcgaaaaagc gatcaacgct aaaaaagaaa catatgtcta cagctatgat gctgataaaa agacgcttct catgacacag ccaaatggcc |

(continued)

| SEQ ID NO | Nucleotide or amino acid sequence |
|---|---|
|  | ggaaagtaca gtatggctat aatgaagcgg gaaatccgat tcaggtcatt gatgatgcgg aaggggttaaa aattacaacg aatacaaagt atgaaggcaa caatgttgta gaagatgtag atccaaatga cgtcggcacc ggaaaagcaa cggaaagcta tcaatatgac aaagatggca acgttacgtc ggttaaagat gcatatggaa ctgagacata cgaatacaac aaaaacaacg atgtcacaaa gatgaaggat accgagggca atgtcacgga tattgcttat gacggactgg atgctgtatc cgaaaccgat cagagcggaa aatcatcgtc agcagctgtt tatgataaat atggaaacca gattcaatcg agcaaagatt tatcagcttc aactaatatt ttgaaagatg gaagctttga agctcaaaaa tccggctgga atctgactgc ctctaaagac aggcgcaaaa tttcagtcat cgccgataaa agcggagttc tttccggatc aaaggcattg gaggtcttga gccaatcaac gtctgccgga acagatcatg ggtattcgtc agcaacacaa acagttgagc tggagccgaa tacaacctat acattaagcg ggaaaatcaa aacagatctt gcgaaatcaa gagcctattt taacattgat ttgcgagaca agatcaaaa acgaattcaa tggattcaca atgaatacag tgcattagcc ggaaaaaatg actggacaaa acggcaaatc acttttacga ctccagcgaa tgcaggcaaa gctgttgtgt atatggaagt cgatcataaa gataaagatg gcaaaggaaa agcatggttt gatgaagtcc agctggaaaa aggcgaagta tcatcaagct ataacccagt tcaaaacagc agcttcacat cagcaacgga aaactggaat gtaagcggag catccgttga ttctgaagaa ggctttaatg atgatgtatc attaaaagct gccccggacca gtgcgtccca agccggatca gtaacaaaac aaaccgttgt cttagggcaa agcgcaaatg acaaaccggt ttatctcaca ctgacaggaa tgtcgaaagc aagcagcgtc aaatttacag atgaaaaaga ctattctctg caggcgaatg tcacttacgc tgacggcagc acaggcatct acaatgcgaa attcccttct ggaacacagg aatggaaccg agcagctgtc gtcatcccta aaacaaagcc gatcaataaa gtcgatatca gtattctgtt ccaaaagagt gcgacaggca cagtctggtt tgatgatatc cgtttgattg aaggaagcct tctgactaaa agcacgtatg acagtaatgg caactatgtg accaaggaag aggacgaact aggttacgcc acttcaactg attacgatga aactggtaag aaaacgtctg aaacagatgc aaaaggtgaa aaaacaacct atacgtatga tcaggcagac caattgacaa acatgacgct ctctaacggc acgtctatcc ttcacagcta tgataaggaa ggcaatgagg taagcaaaac catccgggca ggagcagatc aaacctataa attcgaatat gatgtcatgg gcaagcttgt caaaacaact gaccctctcg gcaatgtgtt agcaagcgaa tatgatgcca acagcaacct gaccaaaacc atttcaccaa atggcaacga agtgtctctt tcttatgatg aacagaccg ggtgaaatca aagtcgtata acggtacaga aaagtacatt ttcacgtatg acaaaaatgg aaatgaaacg tcggtcgtca ataaagagca gaacaccaca aagaaacgga catttgacaa caaaaaccgt ttaaccgaac tgacagaccg cggcggcagc caaacttgga catatcctag tgactccgat aagctgaaaa ccttctcgtg gattcatggg gaccaaaaag gaacgaatca atttacgtat aacaagctgg atcaaatgat tgagatgaaa gacagtacat cctcatattc atttgattat gatgaaaatg gaaacgtcca aacctttatt acgggcaacg gcggcggaac aagttttttca tatgacgaaa gaaaccttgt aagctcgctg catatcggtg acaaaaacgg cggagacatt ctgacggaaa gctatgaata tgatgccaac ggcaaccgca caacgataaa cagctcagca agcggcaagg tgcaatatga gtatgggaag ctgaatcagc ttgtcaaaga aacccatgaa gacggtactg ttattgaata cacatatgat ggattcggaa accggaaaac cgttacaact ataaaagacg gctcaagcaa aacggtgaac gcttccttca acatcatgaa ccagctgaca aaagtcaatg atgaaagcat ctcttacgat |

| SEQ ID NO | Nucleotide or amino acid sequence |
|---|---|
| | aaaaacggca accgcacatc cgacggcaaa ttcacatata cgtgggatgc agaagataac ctgaccgccg ttaccaaaaa aggcgaagac aagccattcg caacatataa gtatgacgaa aaaggaaaca gaatccaaaa aaccgtcaac ggaaaagtca cgaactactt ctacgacgga gacagcttga acgtcttgta tgaaacagac gcggacaaca acgtcacaaa atcatacacg tacggagaca gcggccagct gttatcctac acagaaaatg gtaagaaata tttctatcac tacaacgctc acggcgacat catcgcgatc tcagacagca ctggaaaaac cgttgccaag tatcaatacg acgcatgggg aaatccaaca aaaaccgaag caagcgacga agtaaaagac aatcgctacc gctacgcagg ctaccaatac gacgaagaaa ccggcctgta ctatctcatg gcccgctact acgagccaag aaacggcgtg ttcctatcgc tcgacccaga cccgggcagc gacggagatt cattggatca gaatgggtat gcttatggaa ataataaccc agtgatgaat gttgatccgg acgggcattg ggtatggctt gtggtaaatg ccggatttgc agcctatgat ggatataagg catataaatc aggtaaagga tggaaaggtg ctgcttgggc agctgcttct aattttggtc ctgggaagat atttaagggt gctagtcgcg cctataagtt tacgaagaag gcagttaaaa tcacaggtca tacaagacat ggacttaatc aatctatagg aagaaatgga ggccgtggag tcaatttaag ggccaaactg aatgctgtgc gaagccctaa aaaagtaata aaacaaccta atggggcaac gaaatatgtt ggaaaaaaag ctacagttgt tttaaataaa aggggtaaag taatcacagc ttatggaagt tcaagagcta agggttctaa acacgtattc catacacacg gaaaaggaaa taaatctaag agaaggcgtt aataaggtta agcgagggg ggatgacaac taccctcact taaactttaa atgaggagga aatatggcca aaataaaaga tgattgtata gaacttgaat taacaccgag aaggtatcaa gagttggacg atgatccatt tatttatct gtatttgaat tgttggaaaa taagaaagcc gtcgttcgcg attttctgc tgttttatta gaaagtgaat ataaggtgtt gatatctggg atcgaaacta tgataaaagg aaatcaagac agtatcagtt tagagactat tgaaccattc ttatttctta gtattgatca agaaaacgga aattacagaa ttaagattaa aattatattt gatgattata agaaagtaa gtcgaactcc aacttgttcg aaattaattg taatgaagaa aagctagagt cttttgtgac cgctttaaaa ttaaatcttg aaaatacaaa aaatccttct aaacttcctt aattataaat aatttctgca ttggataaaa atggggcatt tattgatgga acattaaata agagatgtta ccatcctctc ttatttgtat gacaaaagaa tctaaaaata tagaattgat tgggatgaag aggatttgcc aaaaaggttc ccagcgtttt gtgaaaagcc cactgggtga gttttaagca gttgaagata aagcttgtat cgataagcga tacaagcttt tttagaacaa atctaaacat gaacgacaac gaaaacaaga gaatgatgaa tcaatttaaa agaagttgag aaattgacag cggagggaat gcggctcatg caaaaaagat tgatcgaaaa actcgccgag aataaaagac gtttattgat gagcgaaatc aaagaaaaac tagtcccttg tgcagaaaga ggcttcactt atcgttttgc tgatctggcg caaagcaggc aattgcttga ctggttttta tcaaattcaa taccagccaa cacccgtgag gttgatgccc tcgaaaaaac ggaagcgaaa cagctgatct ggaaaatatc tcagtcatat gttgcttacg atgaccggga cgttctgctg tttcatcaat acagcagcga aattggtgcg ttagtttgtt cttttggaaa atgtctcgag caccttgact gcttattgga gtgtattgag ttcgatgaag ggattctcaa tcatagtttt attttggttg agcctaagtg ccagtttggt ttatgtatat gcacacggaa atacggatgt gagcttgttt attggtagat tggaaacctt ttggaacata tcttttacgg aaaaagcttc aatatctaaa tatcagtacg gtgctttgca tactaataaa aaatcattta gtacttgaat acgtggtgat caaattgagc gagacaaatc taatagaatg cataaataaa atcaaaagtg tcttaaatgg acaaacaaca agagaagaag tgtcggattg ggctggaact tatgtttatg cggatgaccc |

(continued)

| SEQ ID NO | Nucleotide or amino acid sequence |
|---|---|
| | cgaagtagaa gacgatagag tctgggatat gttaatcctt ctaagtggga ttgatttgaa agattcatca gaaacttatt tgcattcaac agatgattta aatgattgga tcaaacaata tacggagtaa tttgtttct gcttggtggc aaggcgttat tgtttaaaaa gaggcagggg atgacaatgt atcaaacgat taatgaatgg gttgattata tagataaggg gtgcagtgtc cttcatttag attttaatgt gtttaaaaaa gaactctcac ttgatattaa ggttttttgaa gatgaaggtg aatatacgca taaaatttca tttcaaaatg tggcatccgc ttattatagt gcagatgttg gcgacatgag attggaaaaa atagatccgg aagaatacaa ttggcaagta tttgagatga gttatcatcc agaaggaatc gggaatctat caaattctat aattccggag tatcaatcaa atgctaactt tcttattgac atgaacagaa tgctgattgc tatagaagct gaaacagttc gctttgatga tcaatcgttt tatgcatacc aattaaacac taaatgattt aaggctgaat tggaaagtag atttatccca aaacgattaa tgaaactagg ttgtccgtta ctaatagcta ataaatggtt tattcttatc atttcatttc gaacctagga gcataaccaa tgaaaaaatt aaaattgaat aaaatcatca acgagaacac ccaagattgt tttttccatg ctgatccaca ggggcgtgtc tacattggaa aagggttgaa aggtggtatc accattacaa tctatgatcc ttccggtgat gttcgggatt ttagagtgaa agagcagatt tcatacagtg atattctgct ctttcaacaa tgtgcagatg gctatgtctt cgtttattac gaatcgtacg agccgaaggt aaaggttttt tcagaagagg gaagggtgaa aagtgttttt cacttgcctt caggggtttc ctgctgtctt ttggattaga aacaacagct ttgggtcggt ttatgtgatg aagggattta tcatgatgag aatcccaacg gccatagtgt gtggtgtttt acgttagatg gtcagcagaa ggaaatacac attgatagaa agcttgaaga aatggatgcg cctgctgtag atgattgtta tggacttgca gaaaaaaacg gcttgattta tatgctgtat tacggagatg cagtcattgc cgcatttgat gaaacggtcg tgagacgggt gtggattctg tcggataaag agccggactt agaagcattt gatcagctcg cgatatccga agaaggtttt ttggttggga cgttcaattg ttacagtgca gaccttaagt cgtctcttta tttgatttct gccgatgtga gtcaagagat ggaagagata acatgctgtg attcgaacgg tgaaggccta gatgttcaac agctgaaact gacaagttac gctgtttacg cagtggcctc ttctggtgtt tataagcagg atatccaata aggtgtaggg atgagattat gatgaattgg cgcgctttaa gtcagacaaa acaagaccgc atctggtcag aagtgaacaa gatc |
| **3** (16s rRNA target specific primers 27F) | agagtttgatcctggctcag |
| **4** (16s rRNA target specific primers R357) | ctgctgcctyccgta |

## REFERENCES:

Throughout this application, various references describe the state of the art to which this invention pertains. The disclosures of these references are hereby incorporated by reference into the present disclosure.

1. Nakatsuji T, Cheng JY, and Gallo RL. Mechanisms for control of skin immune function by the microbiome. Curr Opin Immunol. 2021;72:324-30.
2. Belkaid Y, and Tamoutounour S. The influence of skin microorganisms on cutaneous immunity. Nat Rev Immunol. 2016;16(6):353-66.

3. Bukhari S, Mertz AF, and Naik S. Eavesdropping on the conversation between immune cells and the skin epithelium. Int Immunol. 2019;31(7):415-22.

4. Patra V, Byrne SN, and Wolf P. The Skin Microbiome: Is It Affected by UV-induced Immune Suppression? Front Microbiol. 2016;7:1235.

5. Patra V, Wagner K, Arulampalam V, and Wolf P. Skin Microbiome Modulates the Effect of Ultraviolet Radiation on Cellular Response and Immune Function. iScience. 2019; 15:211-22.

6. Hart PH, and Norval M. The Multiple Roles of Urocanic Acid in Health and Disease. J Invest Dermatol. 2021;141(3):496-502.

7. Kammeyer A, Teunissen MB, Pavel S, de Rie MA, and Bos JD. Photoisomerization spectrum of urocanic acid in human skin and in vitro: effects of simulated solar and artificial ultraviolet radiation. Br J Dermatol. 1995;132(6):884-91.

8. McLoone P, Simics E, Barton A, Norval M, and Gibbs NK. An action spectrum for the production of cis-urocanic acid in human skin in vivo. J Invest Dermatol. 2005;124(5):1071-4.

9. Kammeyer A, Pavel S, Asghar SS, Bos JD, and Teunissen MB. Prolonged increase of cis-urocanic acid levels in human skin and urine after single total-body ultraviolet exposures. Photochem Photobiol. 1997;65(3):593-8.

10. Moodycliffe AM, Norval M, Kimber I, and Simpson TJ. Characterization of a monoclonal antibody to cis-urocanic acid: detection of cis-urocanic acid in the serum of irradiated mice by immunoassay. Immunology. 1993;79(4):667-72.

11. De Fabo EC, and Noonan FP. Mechanism of immune suppression by ultraviolet irradiation in vivo. I. Evidence for the existence of a unique photoreceptor in skin and its role in photoimmunology. J Exp Med. 1983;158(1):84-98.

12. Barresi C, Stremnitzer C, Mlitz V, Kezic S, Kammeyer A, Ghannadan M, et al. Increased sensitivity of histidinemic mice to UVB radiation suggests a crucial role of endogenous urocanic acid in photoprotection. J Invest Dermatol. 2011;131(1):188-94.

13. Walterscheid JP, Nghiem DX, Kazimi N, Nutt LK, McConkey DJ, Norval M, et al. Cis-urocanic acid, a sunlight-induced immunosuppressive factor, activates immune suppression via the 5-HT2A receptor. Proc Natl Acad Sci USA. 2006;103(46):17420-5.

14. Garssen J, Norval M, Crosby J, Dortant P, and Van Loveren H. The role of urocanic acid in UVB-induced suppression of immunity to Trichinella spiralis infection in the rat. Immunology. 1999;96(2):298-306.

15. Moodycliffe AM, Kimber I, and Norval M. The effect of ultraviolet B irradiation and urocanic acid isomers on dendritic cell migration. Immunology. 1992;77(3):394-9.

16. Noonan FP, and De Fabo EC. Immunosuppression by ultraviolet B radiation: initiation by urocanic acid. Immunol Today. 1992;13(7):250-4.

17. Yoon SH, Ha SM, Kwon S, Lim J, Kim Y, Seo H, et al. Introducing EzBioCloud: a taxonomically united database of 16S rRNA gene sequences and whole-genome assemblies. Int J Syst Evol Microbiol. 2017;67(5):1613-7.

18. Hug DH, Dunkerson DD, and Hunter JK. The degradation of L-histidine and trans- and cis-urocanic acid by bacteria from skin and the role of bacterial cis-urocanic acid isomerase. J Photochem Photobiol B. 1999;50(1):66-73.

19. Bender RA. Regulation of the histidine utilization (hut) system in bacteria. Microbiol Mol Biol Rev. 2012;76(3):565-84.

20. Yates AD, Allen J, Amode RM, Azov AG, Barba M, Becerra A, et al. Ensembl Genomes 2022: an expanding genome resource for non-vertebrates. Nucleic Acids Res. 2022;50(D1):D996-D1003.

21. Bonneville M, Chavagnac C, Vocanson M, Rozieres A, Benetiere J, Pernet I, et al. Skin contact irritation conditions the development and severity of allergic contact dermatitis. J Invest Dermatol. 2007;127(6):1430-5.

22. Willmott T, Campbell PM, Griffiths CEM, O'Connor C, Bell M, Watson REB, et al. Behaviour and sun exposure in holidaymakers alters skin microbiota composition and diversity. Frontiers in Aging. 2023;4.

23. Matallana-Surget S, Meador JA, Joux F, and Douki T. Effect of the GC content of DNA on the distribution of UVB-induced bipyrimidine photoproducts. Photochem Photobiol Sci. 2008;7(7):794-801.

24. Oppezzo O, Costa C, and Pizarro R.. Effects of ultraviolet A radiation on survival and growth of Gram negative bacteria. Trends in Photochemistry and Photobiology. 2011;13:37-50.

25. Gläser R, Navid F, Schuller W, Jantschitsch C, Harder J, Schröder JM, et al. UV-B radiation induces the expression of antimicrobial peptides in human keratinocytes in vitro and in vivo. J Allergy Clin Immunol. 2009;123(5):1117-23.

26. Felton S, Navid F, Schwarz A, Schwarz T, Gläser R, and Rhodes LE. Ultraviolet radiation-induced upregulation of antimicrobial proteins in health and disease. Photochem Photobiol Sci. 2013;12(1):29-36.

27. Schauber J, and Gallo RL. Antimicrobial peptides and the skin immune defense system. J Allergy Clin Immunol. 2008;122(2):261-6.

28. Patra V, Bordag N, Clement Y, Köfeler H, Nicolas J-F, Vocanson M, et al. Ultraviolet exposure regulates skin metabolome based on the microbiome. Scientific Reports. 2023;13(1):7207.

29. Yeh C-Y, Su S-H, Tan YF, Tsai T-F, Liang P-H, Kelel M, et al. PD-L1 Enhanced by cis-Urocanic Acid on Langerhans Cells Inhibits Yγ4+ γδT17 Cells in Imiquimod-Induced Skin Inflammation. Journal of Investigative Dermatology. 2023;143(8):1449-60.

30. Boxberger M, Cenizo V, Cassir N, and La Scola B. Challenges in exploring and manipulating the human skin microbiome. Microbiome. 2021;9(1):125.

31. Rosenbaum MD, VandeWoude S, and Johnson TE. Effects of cage-change frequency and bedding volume on mice and their microenvironment. J Am Assoc Lab Anim Sci. 2009;48(6):763-73.

32. Naik S, Bouladoux N, Linehan JL, Han SJ, Harrison OJ, Wilhelm C, et al. Commensal-dendritic-cell interaction specifies a unique protective skin immune signature. Nature. 2015; 520(7545):104-8.

33. Lei YM, Sepulveda M, Chen L, Wang Y, Pirozzolo I, Theriault B, et al. Skin-restricted commensal colonization accelerates skin graft rejection. JCI Insight. 2019;5(15).

34. Klymiuk I, Bambach I, Patra V, Trajanoski S, and Wolf P. 16S Based Microbiome Analysis from Healthy Subjects' Skin Swabs Stored for Different Storage Periods Reveal Phylum to Genus Level Changes. Front Microbiol. 2016;7:2012.

35. Yap M, O'Sullivan O, O'Toole PW, and Cotter PD. Development of sequencing-based methodologies to distinguish viable from non-viable cells in a bovine milk matrix: A pilot study. Front Microbiol. 2022;13:1036643.

36. Patra V, Woltsche N, Cerpes U, Bokanovic D, Repelnig M, Joshi A, et al. Persistent neutrophil infiltration and unique ocular surface microbiome typify dupilumab-associated conjunctivitis in patients with atopic dermatitis. Ophthalmology Science.

37. Bolyen E, Rideout JR, Dillon MR, Bokulich NA, Abnet CC, Al-Ghalith GA, et al. Reproducible, interactive, scalable and extensible microbiome data science using QIIME 2. Nat Biotechnol. 2019;37(8):852-7.

38. Callahan BJ, McMurdie PJ, Rosen MJ, Han AW, Johnson AJ, and Holmes SP. DADA2: High-resolution sample inference from Illumina amplicon data. Nat Methods. 2016;13(7):581-3.

39. Quast C, Pruesse E, Yilmaz P, Gerken J, Schweer T, Yarza P, et al. The SILVA ribosomal RNA gene database project: improved data processing and web-based tools. Nucleic Acids Res. 2013;41(Database issue):D590-6.

40. Chen L, Reeve J, Zhang L, Huang S, Wang X, and Chen J. GMPR: A robust normalization method for zero-inflated count data with application to microbiome sequencing data. PeerJ. 2018;6:e4600.

41. Love MI, Huber W, and Anders S. Moderated estimation of fold change and dispersion for RNA-seq data with DESeq2. Genome Biol. 2014;15(12):550.

## Claims

1. A method of treating an inflammatory skin disease in a subject in need thereof comprising administering to the subject a therapeutically effective amount of an urocanase inhibitor.

2. The method of claim 1 wherein said administration of urocanase inhibitor is associated with phototherapy.

3. The method according to claim 2 wherein phototherapy is UVB phototherapy.

4. The method according to claim 2 or 3 wherein the urocanase inhibitor is administered prior to phototherapy.

5. The method according to anyone of claim 1 to 4 wherein urocanase inhibitor is administered topically.

6. The method according to anyone of claim 1 to 3, wherein the inflammatory skin disease is selected from the group consisting of dermatitis, atopic dermatitis, Netherton syndrome, SAM, SAMEC syndromes, psoriasis, polymorphic light eruption and vitiligo.

7. The method according to anyone of claim 1 to 6, which comprises a first step for determining whether the subject suffering from an inflammatory skin disease has Hut+ skin bacterial species selected from the list consisting of Pseudomonas fluorescens, Pseudomonas fragi, Staphylococcus epidermidis; Pseudomonas azotoformans, Flavobacterium succinicans, Corynebacterium tuberculostearicum, Staphylococcus lentus Staphylococcus xylosus, Parabacteroides distasonis ; Alistipes finegoldii, Staphylococcus caprae.

8. The method of claim 7 wherein the first step consists in detecting the skin bacterial species that is Staphylococcus epidermidis.

9. The method according to anyone of claim 1 to 8 wherein the urocanase inhibitor is 1)

   1) an inhibitor of urocanase activity selected from the list consisting of small organic molecule, antibody, aptamer, polypeptide, and/ or

2) an inhibitor of urocanase gene expression selected from the list consisting of antisense oligonucleotide, siRNA, ribozymes.

10. The method according to anyone of claim 9, wherein the inhibitor of urocanase activity is glycineglycine.

Figure 1A, B, C, D and E

**A**

8h

**B**

24h

- 1. Acidobacteria
- 2. Actinobacteria
- 3. Bacteroidetes
- 4. Elusimicrobia
- 5. Epsilonbacteraeota
- 6. Firmicutes
- 7. Fusobacteria
- 8. Gemmatimonadetes
- 9. Patescibacteria
- 10. Proteobacteria
- 11. Tenericutes
- 12. Verrucomicrobia

**Figure 2 A and B**

**C** Baseline vs after 8h

Increase in abundance [ASV]

cisUCA 45    9    UVB 45

**D** Baseline vs after 24h

Increase in abundance [ASV]

cisUCA 15    3    UVB 44

**E**

Decrease in abundance [ASV]

cisUCA 2    UVB 13

**F**

Decrease in abundance [ASV]

cisUCA 25    6    UVB 41

**Figure 2C, D E and F**

Figure 3A, B C and D

**E**

Glycylglycine (glygly) inhibition- *Staphylococcus epidermidis*

**=**

Glycylglycine (glygly) inhibition- *Corynebacterium singulare*

**Figure 3E and F**

Figure 4A B and C

**A**

**B**

**Figure 5A and B**

**Figure 6A and B**

**Figure 7 A and B**

Figure 7 C and D

Figure 8

Europäisches
Patentamt

European
Patent Office

Office européen
des brevets

# EUROPEAN SEARCH REPORT

**Application Number**

EP 24 30 6213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Vijay Kumar Patra: "ESDR231 - cis-urocanic acid mediated cutaneous immune suppression is controlled by microbiome.", , 25 September 2021 (2021-09-25), XP002812793, Retrieved from the Internet: URL:https://www.morressier.com/o/event/610 80a2c4517da001280e092/article/61081ff8bc98 1037240fe473 [retrieved on 2024-12-19] * the whole document * | 1-10 | INV. A61K38/05 A61K31/198 A61K31/7105 A61N5/00 A61P17/00 |
| Y | HUG DANIEL H ET AL: "The degradation of L-histidine and tram-and cis-urocanic acid by bacteria from skin and the role of bacterial cis-urocanic acid isomerase", JOURNAL OF PHOTOCHEMISTRY AND PHOTOBIOLOGY B: BIOLOGY, vol. 50, 1 May 1999 (1999-05-01), pages 66-73, XP093235818, * abstract * | 1-10 | |
| Y | SINGER SEBASTIAN ET AL: "Phototherapy", JDDG. JOURNAL DER DEUTSCHEN DERMATOLOGISCHEN GESELLSCHAFT, [Online] vol. 16, no. 9, 1 September 2018 (2018-09-01), pages 1120-1129, XP055967925, DE ISSN: 1610-0379, DOI: 10.1111/ddg.13646 Retrieved from the Internet: URL:https://api.wiley.com/onlinelibrary/td m/v1/articles/10.1111%2Fddg.13646> [retrieved on 2024-12-19] * abstract * -/-- | 1-10 | TECHNICAL FIELDS SEARCHED (IPC) A61K A61N A61P |

The present search report has been drawn up for all claims

| Place of search | Date of completion of the search | Examiner |
|---|---|---|
| Munich | 1 January 2025 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| Europäisches Patentamt European Patent Office Office européen des brevets | **EUROPEAN SEARCH REPORT** | **Application Number** EP 24 30 6213 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | & PATRA V. ET AL: "1052 Urocanase-positive bacteria in the skin regulate the immunomodulatory properties of the UV photoproduct, cis-urocanic acid", JOURNAL OF INVESTIGATIVE DERMATOLOGY, 1 May 2023 (2023-05-01), page S180, XP093236014, DOI: :10.1016/j.jid.2023.03.1063 Retrieved from the Internet: URL:https://www.sciencedirect.com/science/article/pii/S0022202X23012381?via%3Dihub [retrieved on 2024-12-19] | 1-10 | |
| Y | * abstract * | 1-10 | |
| Y | Antti Laurema I. ET AL: "Topical cis-urocanic acid suppresses both induction and elicitation of contact hypersensitivity in BALB/C mice", Acta Derm Venerol, 1 July 1995 (1995-07-01), pages 272-275, XP093235799, Stockholm [retrieved on 2024-12-19] * abstract * * Results * | 1-10 | TECHNICAL FIELDS SEARCHED (IPC) |

-/--

The present search report has been drawn up for all claims

| Place of search | Date of completion of the search | Examiner |
|---|---|---|
| Munich | 1 January 2025 | Böhmerova, Eva |

EPO FORM 1503 03.82 (P04C01)

Europäisches
Patentamt

European
Patent Office

Office européen
des brevets

**EUROPEAN SEARCH REPORT**

**Application Number**

EP 24 30 6213

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PELTONEN J ET AL: "Three Randomised Phase I/IIa Trials of 5% Cis-urocanic Acid Emulsion Cream in Healthy Adult Subjects and in Patients with Atopic Dermatitis", ACTA DERMATO-VENEREOLOGICA., 1 January 2014 (2014-01-01), pages 415-420, XP093235803, United Kingdom ISSN: 0001-5555, DOI: 10.2340/00015555-1735 Retrieved from the Internet: URL:https://www.medicaljournals.se/acta/content_files/files/pdf/94/4/4049.pdf [retrieved on 2024-12-19] * abstract * * figure 2 * | 1-10 | |
| A | US 5 582 817 A (OTSU YOSHIRO [JP] ET AL) 10 December 1996 (1996-12-10) * claims 1,8,16,28,37,46 * | 1-10 | |
| | | | TECHNICAL FIELDS SEARCHED (IPC) |
| A | US 10 806 721 B2 (DRUG DELIVERY SOLUTIONS [DK]; DRUG DELIVERY SOLUTIONS APS [DK]) 20 October 2020 (2020-10-20) * claims 1,2, 14 * | 1-10 | |

The present search report has been drawn up for all claims

| Place of search | Date of completion of the search | Examiner |
|---|---|---|
| Munich | 1 January 2025 | Böhmerova, Eva |

EPO FORM 1503 03.82 (P04C01)

page 3 of 3

## ANNEX TO THE EUROPEAN SEARCH REPORT
## ON EUROPEAN PATENT APPLICATION NO.

EP 24 30 6213

This annex lists the patent family members relating to the patent documents cited in the above-mentioned European search report.
The members are as contained in the European Patent Office EDP file on
The European Patent Office is in no way liable for these particulars which are merely given for the purpose of information.

01-01-2025

| Patent document cited in search report | | Publication date | Patent family member(s) | | Publication date |
|---|---|---|---|---|---|
| US 5582817 | A | 10-12-1996 | AU | 3462993 A | 01-09-1993 |
| | | | CA | 2107461 A1 | 04-08-1993 |
| | | | EP | 0583479 A1 | 23-02-1994 |
| | | | TW | 233264 B | 01-11-1994 |
| | | | US | 5582817 A | 10-12-1996 |
| | | | WO | 9314748 A1 | 05-08-1993 |
| US 10806721 | B2 | 20-10-2020 | AU | 2017283511 A1 | 17-01-2019 |
| | | | AU | 2020202785 A1 | 14-05-2020 |
| | | | BR | 112018076229 A2 | 26-03-2019 |
| | | | CA | 3026620 A1 | 21-12-2017 |
| | | | CN | 109414430 A | 01-03-2019 |
| | | | CN | 115671098 A | 03-02-2023 |
| | | | EP | 3471720 A1 | 24-04-2019 |
| | | | EP | 4397368 A2 | 10-07-2024 |
| | | | JP | 7019684 B2 | 15-02-2022 |
| | | | JP | 7220316 B2 | 09-02-2023 |
| | | | JP | 2019518084 A | 27-06-2019 |
| | | | JP | 2022048311 A | 25-03-2022 |
| | | | JP | 2022187022 A | 15-12-2022 |
| | | | KR | 20190020022 A | 27-02-2019 |
| | | | KR | 20220143149 A | 24-10-2022 |
| | | | KR | 20240100484 A | 01-07-2024 |
| | | | RU | 2018146294 A | 17-07-2020 |
| | | | US | 2019321336 A1 | 24-10-2019 |
| | | | US | 2021000796 A1 | 07-01-2021 |
| | | | WO | 2017215723 A1 | 21-12-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6566135 B
- US 6566131 B
- US 6365354 B
- US 6410323 B
- US 6107091 A
- US 6046321 A
- US 5981732 A
- US 6573099 B

- US 6506559 B
- WO 0136646 A
- WO 9932619 A
- WO 0168836 A
- US 4946778 A
- US 5225539 A
- US 4816397 A
- WO 1999048470 A

**Non-patent literature cited in the description**

- **PALMER CNA et al.** *Nat Genet*, 2006, vol. 38, 441-6
- **CORK MJ et al.** *J Invest Dermatol*, 2009, vol. 129, 1892-908
- **ESPINÓS C et al.** *Journal of Medical Genetics.*, June 2009, vol. 46 (6), 407-11
- **RETEY J**. *Arch. Biochem. Biophys.*, 1994, vol. 314, 1-16
- **KESSLER D** ; **RETEY J** ; **SCHULZ GE**. *J. Mol. Biol.*, 2004, vol. 342, 183-194
- **ESPINOS C et al.** *J. Med. Genet.*, 2009, vol. 46, 407-411
- **BENDER, R.A**. Regulation of the histidine utilization (hut) system in bacteria. *Microbiol Mol Biol Rev*, 2012, vol. 76, 565-584
- **VLADIMIR HOLÁŇ** ; **LUCIA KUFFOVÁ** ; **ALENA ZAJÍCOVÁ** ; **MAGDALÉNA KRULOVÁ** ; **MARTIN FILIPEC** ; **PETR HOLLER** ; **ALEXANDER JANČÁREK**. Urocanic Acid Enhances IL-10 Production in Activated CD4+ T Cells. *J Immunol*, 1998, vol. 161 (7), 3237-3241
- **F M RATTIS 1** ; **J PÉGUET-NAVARRO** ; **P COURTELLEMONT** ; **G REDZINIAK** ; **D SCHMITT**. Cis-urocanic acid failed to affect in vitro human Langerhans cell allostimulatory function. *Photochem Photobiol.*, November 1995, vol. 62 (5), 914-6
- **R.H.A. Plimmer.** July 2008, 1908
- **DUNN, MAX S. et al.** *Shaking with alkaliand other synthesis methods have been reported*, 1932
- Journal of Biological Chemistry. American Society for Biochemistry. *Molecular Biology*, vol. 99 (1), 217-220
- **SMITH, MARSHALL E** ; **WOODS HOLE et al.** The Biological Bulletin. *MA: Marine Biological Laboratory.*, 1949, vol. 96 (3), 233-237
- **HUNTER JK et al.** *Pept Res.*, May 1989, vol. 2 (3), 240-5

- **NAKATSUJI T** ; **CHENG JY** ; **GALLO RL**. Mechanisms for control of skin immune function by the microbiome. *Curr Opin Immunol.*, 2021, vol. 72, 324-30
- **BELKAID Y** ; **TAMOUTOUNOUR S**. The influence of skin microorganisms on cutaneous immunity. *Nat Rev Immunol.*, 2016, vol. 16 (6), 353-66
- **BUKHARI S** ; **MERTZ AF** ; **NAIK S**. Eavesdropping on the conversation between immune cells and the skin epithelium. *Int Immunol.*, 2019, vol. 31 (7), 415-22
- **PATRA V** ; **BYRNE SN** ; **WOLF P**. The Skin Microbiome: Is It Affected by UV-induced Immune Suppression?. *Front Microbiol.*, 2016, vol. 7, 1235
- **PATRA V** ; **WAGNER K** ; **ARULAMPALAM V** ; **WOLF P**. Skin Microbiome Modulates the Effect of Ultraviolet Radiation on Cellular Response and Immune Function. *iScience*, 2019, vol. 15, 211-22
- **HART PH** ; **NORVAL M**. The Multiple Roles of Urocanic Acid in Health and Disease. *J Invest Dermatol.*, 2021, vol. 141 (3), 496-502
- **KAMMEYER A** ; **TEUNISSEN MB** ; **PAVEL S** ; **DE RIE MA** ; **BOS JD.** Photoisomerization spectrum of urocanic acid in human skin and in vitro: effects of simulated solar and artificial ultraviolet radiation. *Br J Dermatol.*, 1995, vol. 132 (6), 884-91
- **MCLOONE P** ; **SIMICS E** ; **BARTON A** ; **NORVAL M** ; **GIBBS NK**. An action spectrum for the production of cis-urocanic acid in human skin in vivo.. *J Invest Dermatol.*, 2005, vol. 124 (5), 1071-4
- **KAMMEYER A** ; **PAVEL S** ; **ASGHAR SS** ; **BOS JD** ; **TEUNISSEN MB**. Prolonged increase of cis-urocanic acid levels in human skin and urine after single total-body ultraviolet exposures. *Photochem Photobiol.*, 1997, vol. 65 (3), 593-8

- **MOODYCLIFFE AM** ; **NORVAL M** ; **KIMBER I** ; **SIMPSON TJ**. Characterization of a monoclonal antibody to cis-urocanic acid: detection of cis-urocanic acid in the serum of irradiated mice by immunoassay. *Immunology*, 1993, vol. 79 (4), 667-72
- **DE FABO EC** ; **NOONAN FP**. Mechanism of immune suppression by ultraviolet irradiation in vivo. I. Evidence for the existence of a unique photoreceptor in skin and its role in photoimmunology. *J Exp Med.*, 1983, vol. 158 (1), 84-98
- **BARRESI C** ; **STREMNITZER C** ; **MLITZ V** ; **KEZIC S** ; **KAMMEYER A** ; **GHANNADAN M et al.** Increased sensitivity of histidinemic mice to UVB radiation suggests a crucial role of endogenous urocanic acid in photoprotection. *J Invest Dermatol.*, 2011, vol. 131 (1), 188-94
- **WALTERSCHEID JP** ; **NGHIEM DX** ; **KAZIMI N** ; **NUTT LK** ; **MCCONKEY DJ** ; **NORVAL M et al.** Cis-urocanic acid, a sunlight-induced immunosuppressive factor, activates immune suppression via the 5-HT2A receptor.. *Proc Natl Acad Sci USA.*, 2006, vol. 103 (46), 17420-5
- **GARSSEN J** ; **NORVAL M** ; **CROSBY J** ; **DORTANT P** ; **VAN LOVEREN H**. The role of urocanic acid in UVB-induced suppression of immunity to Trichinella spiralis infection in the rat.. *Immunology*, 1999, vol. 96 (2), 298-306
- **MOODYCLIFFE AM** ; **KIMBER I** ; **NORVAL M.** The effect of ultraviolet B irradiation and urocanic acid isomers on dendritic cell migration. *Immunology*, 1992, vol. 77 (3), 394-9
- **NOONAN FP** ; **DE FABO EC**. Immunosuppression by ultraviolet B radiation: initiation by urocanic acid.. *Immunol Today.*, 1992, vol. 13 (7), 250-4
- **YOON SH** ; **HA SM** ; **KWON S** ; **LIM J** ; **KIM Y** ; **SEO H et al.** Introducing EzBioCloud: a taxonomically united database of 16S rRNA gene sequences and whole-genome assemblies. *Int J Syst Evol Microbiol.*, 2017, vol. 67 (5), 1613-7
- **HUG DH** ; **DUNKERSON DD** ; **HUNTER JK**. The degradation of L-histidine and trans- and cis-urocanic acid by bacteria from skin and the role of bacterial cis-urocanic acid isomerase. *J Photochem Photobiol B*, 1999, vol. 50 (1), 66-73
- **BENDER RA**. Regulation of the histidine utilization (hut) system in bacteria.. *Microbiol Mol Biol Rev*, 2012, vol. 76 (3), 565-84
- **YATES AD** ; **ALLEN J** ; **AMODE RM** ; **AZOV AG** ; **BARBA M** ; **BECERRA A et al.** Ensembl Genomes 2022: an expanding genome resource for non-vertebrates. *Nucleic Acids Res.*, 2022, vol. 50 (D1), D996-D1003
- **BONNEVILLE M** ; **CHAVAGNAC C** ; **VOCANSON M** ; **ROZIERES A** ; **BENETIERE J** ; **PERNET I et al.** Skin contact irritation conditions the development and severity of allergic contact dermatitis. *J Invest Dermatol.*, 2007, vol. 127 (6), 1430-5
- **WILLMOTT T** ; **CAMPBELL PM** ; **GRIFFITHS CEM** ; **O'CONNOR C** ; **BELL M** ; **WATSON REB et al.** Behaviour and sun exposure in holidaymakers alters skin microbiota composition and diversity. *Frontiers in Aging*, 2023, vol. 4
- **MATALLANA-SURGET S** ; **MEADOR JA** ; **JOUX F** ; **DOUKI T**. Effect of the GC content of DNA on the distribution of UVB-induced bipyrimidine photoproducts. *Photochem Photobiol Sci.*, 2008, vol. 7 (7), 794-801
- **OPPEZZO O** ; **COSTA C** ; **PIZARRO R.** Effects of ultraviolet A radiation on survival and growth of Gram negative bacteria. *Trends in Photochemistry and Photobiology.*, 2011, vol. 13, 37-50
- **GLÄSER R** ; **NAVID F** ; **SCHULLER W** ; **JANTSCHITSCH C** ; **HARDER J** ; **SCHRÖDER JM et al.** UV-B radiation induces the expression of antimicrobial peptides in human keratinocytes in vitro and in vivo. *J Allergy Clin Immunol.*, 2009, vol. 123 (5), 1117-23
- **FELTON S** ; **NAVID F** ; **SCHWARZ A** ; **SCHWARZ T** ; **GLÄSER R** ; **RHODES LE**. Ultraviolet radiation-induced upregulation of antimicrobial proteins in health and disease. *Photochem Photobiol Sci.*, 2013, vol. 12 (1), 29-36
- **SCHAUBER J** ; **GALLO RL**. Antimicrobial peptides and the skin immune defense system. *J Allergy Clin Immunol.*, 2008, vol. 122 (2), 261-6
- **PATRA V** ; **BORDAG N** ; **CLEMENT Y** ; **KÖFELER H** ; **NICOLAS J-F** ; **VOCANSON M et al.** Ultraviolet exposure regulates skin metabolome based on the microbiome. *Scientific Reports*, 2023, vol. 13 (1), 7207
- **YEH C-Y** ; **SU S-H** ; **TAN YF** ; **TSAI T-F** ; **LIANG P-H** ; **KELEL M et al.** PD-L1 Enhanced by cis-Urocanic Acid on Langerhans Cells Inhibits Yγ4+ γδT17 Cells in Imiquimod-Induced Skin Inflammation. *Journal of Investigative Dermatology.*, 2023, vol. 143 (8), 1449-60
- **BOXBERGER M** ; **CENIZO V** ; **CASSIR N** ; **LA SCOLA B.** Challenges in exploring and manipulating the human skin microbiome. *Microbiome*, 2021, vol. 9 (1), 125
- **ROSENBAUM MD** ; **VANDEWOUDE S** ; **JOHNSON TE**. Effects of cage-change frequency and bedding volume on mice and their microenvironment. *J Am Assoc Lab Anim Sci.*, 2009, vol. 48 (6), 763-73
- **NAIK S** ; **BOULADOUX N** ; **LINEHAN JL** ; **HAN SJ** ; **HARRISON OJ** ; **WILHELM C et al.** Commensal-dendritic-cell interaction specifies a unique protective skin immune signature. *Nature*, 2015, vol. 520 (7545), 104-8
- **LEI YM** ; **SEPULVEDA M** ; **CHEN L** ; **WANG Y** ; **PIROZZOLO I** ; **THERIAULT B et al.** Skin-restricted commensal colonization accelerates skin graft rejection. *JCI Insight*, 2019, vol. 5 (15)

- **KLYMIUK I** ; **BAMBACH I** ; **PATRA V** ; **TRAJANOSKI S** ; **WOLF P**. 16S Based Microbiome Analysis from Healthy Subjects' Skin Swabs Stored for Different Storage Periods Reveal Phylum to Genus Level Changes.. *Front Microbiol.*, 2016, vol. 7, 2012
- **YAP M** ; **O'SULLIVAN O** ; **O'TOOLE PW** ; **COTTER PD**. Development of sequencing-based methodologies to distinguish viable from non-viable cells in a bovine milk matrix: A pilot study. *Front Microbiol.*, 2022, vol. 13, 1036643
- **PATRA V** ; **WOLTSCHE N** ; **CERPES U** ; **BOKA-NOVIC D** ; **REPELNIG M** ; **JOSHI A et al.** Persistent neutrophil infiltration and unique ocular surface microbiome typify dupilumab-associated conjunctivitis in patients with atopic dermatitis.. *Ophthalmology Science*
- **BOLYEN E** ; **RIDEOUT JR** ; **DILLON MR** ; **BOKU-LICH NA** ; **ABNET CC** ; **AL-GHALITH GA et al.** Reproducible, interactive, scalable and extensible microbiome data science using QIIME 2. *Nat Biotechnol.*, 2019, vol. 37 (8), 852-7
- **CALLAHAN BJ** ; **MCMURDIE PJ** ; **ROSEN MJ** ; **HAN AW** ; **JOHNSON AJ** ; **HOLMES SP.** DADA2: High-resolution sample inference from Illumina amplicon data. *Nat Methods.*, 2016, vol. 13 (7), 581-3
- **QUAST C** ; **PRUESSE E** ; **YILMAZ P** ; **GERKEN J** ; **SCHWEER T** ; **YARZA P et al.** The SILVA ribosomal RNA gene database project: improved data processing and web-based tools. *Nucleic Acids Res.*, 2013, vol. 41, D590-6
- **CHEN L** ; **REEVE J** ; **ZHANG L** ; **HUANG S** ; **WANG X** ; **CHEN J.** GMPR: A robust normalization method for zero-inflated count data with application to microbiome sequencing data. *PeerJ*, 2018, vol. 6, e4600
- **LOVE MI** ; **HUBER W** ; **ANDERS S.** Moderated estimation of fold change and dispersion for RNA-seq data with DESeq2. *Genome Biol.*, 2014, vol. 15 (12), 550